# EUROPEAN PATENT SPECIFICATION

(11) **EP 0 458 207 B1**
(45) Date of publication and mention of the grant of the patent: **26.03.1997**
(21) Application number: 91108019.0
(22) Date of filing: 17.05.1991
(51) Int. Cl.: C07D 209/12, C07D 209/14, C07D 403/10, C07D 405/06, C07D 413/10, C07D 417/10, A61K 31/40

(54) **Indole derivatives**
Indolderivate
Dérivés d'indole

(30) Priority: 21.05.1990 GB 9011335
(43) Date of publication of application: 27.11.1991
(73) Proprietor: FUJISAWA PHARMACEUTICAL CO., LTD., Osaka-shiOsaka 541 (JP)
(72) Inventor: Okada, Satoshi, Tsukuba-shi,Ibaraki 305 (JP); Sawada, Kozo, Tsukuba-shi,Ibaraki 305 (JP); Kayakiri, Natsuko, Tsukuba-shi,Ibaraki 305 (JP); Saitoh, Yuki, Tsukuba-shi,Ibaraki 305 (JP); Tanaka, Hirokazu, Tsuchiura-shi,Ibaraki 300 (JP); Hashimoto, Masashi, Itabashi-ku,Tokyo 175 (JP)
(74) Representative: Hrabal, Ulrich, Dr.

(56) References cited:
- EP-A- 0 171 037
- US-A- 3 856 967
- EUROPEAN JOURNAL OF MEDICINAL CHEMISTRY - CHIMICA THERAPEUTICA, vol. 10, no. 2, March-April 1975, pages 187-199, Châtenay-Malabry, FR; A. ALLAIS et al.: "Recherche d'analgésiques non narcotiques et d'anti-inflammatoires dans la série des carboxyalcoyl-1 acyl-3 indoles"
- JOURNAL OF MEDICINAL CHEMISTRY, vol. 17, no. 12, 1974, pages 1298-1304, Washington, DC, US; C.W. WHITEHEAD et al.: "Effect of lipophilic substituents on some biological properties of indoles"

## Description

The present invention relates to indole derivatives and a pharmaceutically acceptable salt thereof which have pharmacological activities such as inhibitory activity on testosteron Sa-reductase and the like, to process for preparation thereof, to a pharmaceutical composition comprising the same and to a use of the same for the manufacture of a medicament.

Accordingly, one object of the present invention is to provide novel indole derivatives and a pharmaceutically acceptable salt thereof, which are useful as a testosteron 5a-reductase inhibitor.

Another object of the present invention is to provide process for preparation of said indole derivatives or a salt thereof.

A further object of the present invention is to provide a pharmaceutical composition comprising, as an active ingredient, said indole derivatives or a pharmaceutically acceptable salt thereof.

Still further object of the present invention is to provide a use of said indole derivatives or a pharmaceutically acceptable salt thereof for the manufacture of a medicament such as testosteron 5a-reductase inhibitor useful for treating or preventing testosteron 5a-reductase mediated diseases such as alopecia, acnes, prostatism, and the like in human being or animals.

The indole derivatives of the present invention are novel and can be represented by the formula (I) : 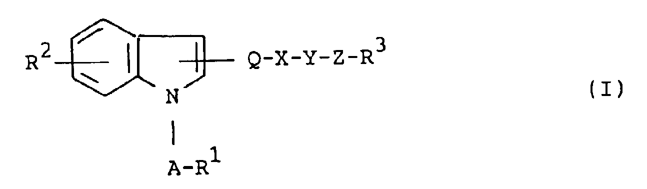 wherein R¹, R², R³, A, Q, X, y and Z are defined as in claim 1.

According to the present invention, the object compound (I) and a salt thereof can be prepared by the following processes. 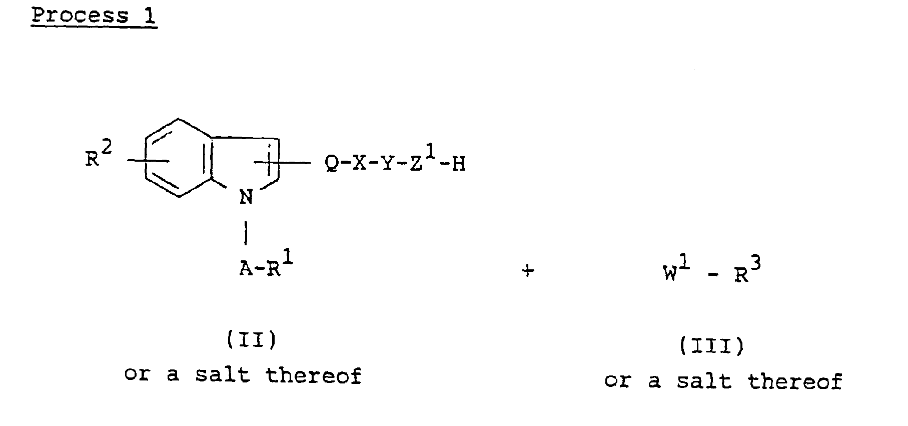 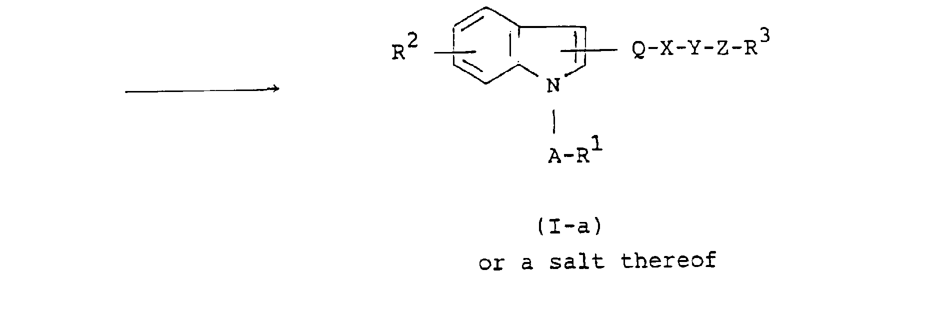 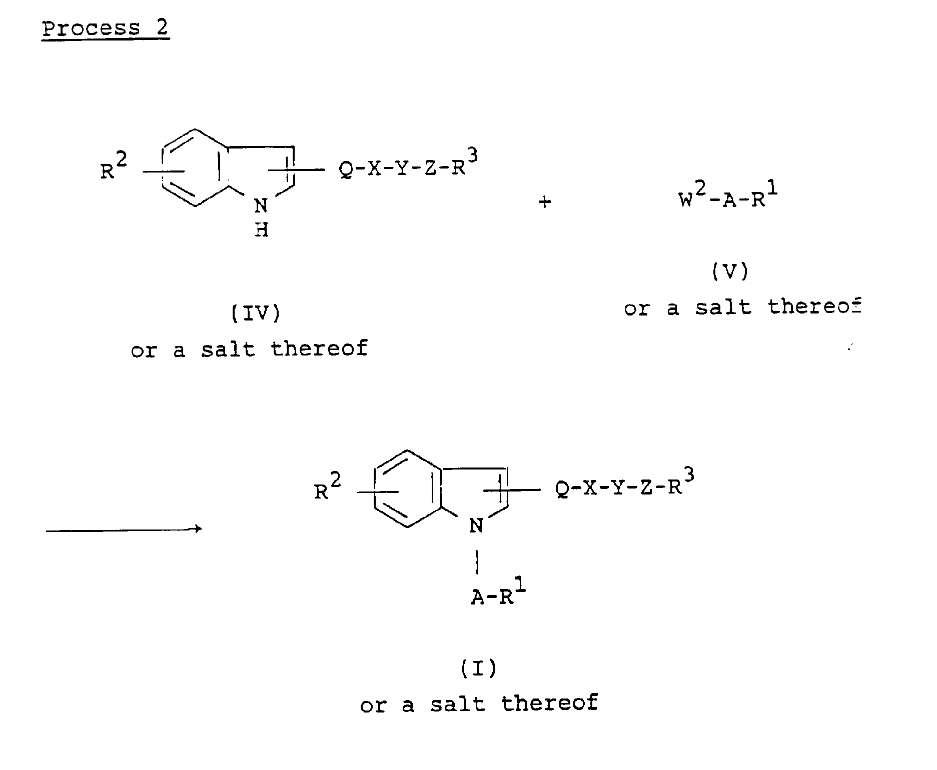 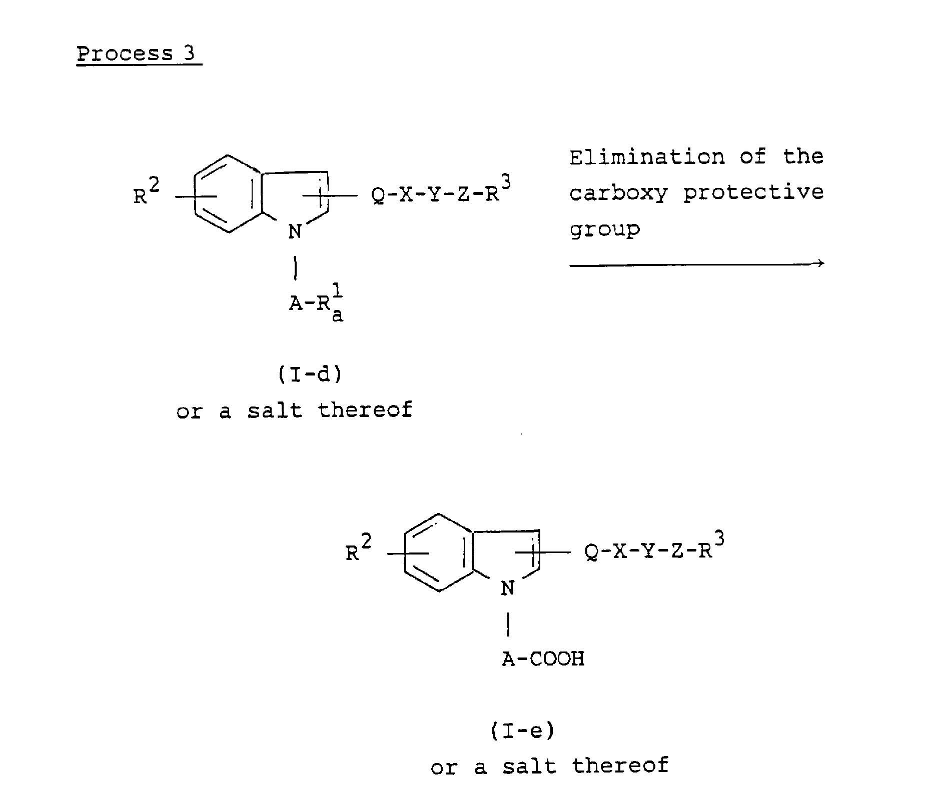 wherein R¹, R², R³, A, Q, X, Y and Z are each as defined above,
is protected carboxy,
W¹, W2 are each acid residue,
Z¹ is -O- or 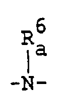
in which R6 is hydrogen or (C₁-C₆) alkyl

Suitable salts of the compounds (I) are conventional non-toxic, pharmaceutically acceptable salt and may include a salt with a base or an acid addition salt such as a salt with an inorganic base, for example, an alkali metal salt (e.g. sodium salt, potassium salt, cesium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.), etc.; an inorganic acid addition salt (e.g. hydrochloride, hydrobromide, sulfate, phosphate, etc.);
an organic carboxylic or sulfonic acid addition salt (e.g. formate, acetate, trifluoroacetate, maleate, tartrate, methanesulfonate, benzenesulfonate, p-toluenesulfonate, etc.); a salt with a basic or acidic amino acid (e.g. arginine, aspartic acid, glutamic acid, etc.); and the like, and the preferable example thereof is an acid addition salt.

With respect to the salt of the compounds (I-a) to (I-k), (II), (III), (IV), (V), (VI), (VII), (VIII), (IX) and (X) in Processes 1 to 10, the suitable examples of the salts of these compounds are to be referred to those as exemplified for the object compound (I).

In the above and subsequent descriptions of the present specification, suitable examples and illustrations of the various definitions which the present invention include within the scope thereof are explained in detail as follows.

Suitable "(C₁-C₆) alkyl" may include straight or branched one, having 1 to 6 carbon atom(s), such as methyl, ethyl, propyl, isopropyl, butyl, isobutyl, t-butyl, pentyl, hexyl, and the like, preferably one having 1 to 4 carbon atoms.

The term "halogen" means fluoro, chloro, bromo and iodo.

Suitable "(C₁-C₆) alkylene" means straight or branched bivalent (C₁-C₆) alkane such as methylene, ethylene, trimethylene, tetramethylene, pentamethylene, hexamethylene, propylene, and the like, which may be substituted by oxo.

Suitable "acid residue" may include halogen (e.g. fluoro, chloro, bromo, iodo), acyloxy (e.g. acetoxy, tosyloxy, mesyloxy, etc.) and the like.

Suitable "(C₂-C₆) alkenylene" may include one having 2 to 6 carbon atoms such as vinylene, propenylene, and the like.

Suitable "aryl which may have suitable substituent(s)" may include a conventional group such as aryl (e.g. phenyl, naphthyl, etc.), substituted aryl, for example, C₁-C₆ alkylaryl (e.g. tolyl, xylyl, mesityl, cumenyl, isobutylphenyl, etc.), haloaryl (e.g. chlorophenyl, etc.) and the like.

Suitable "ar(C₁-C₆)alkyl which may have suitable substituent(s)" may include a conventional group such as ar(C₁-C₆)alkyl (e.g. trityl, benzhydryl, benzyl, phenethyl, naphthylmethyl, etc.), substituted ar(C₁-C₆)alkyl, for example, ar (C₁-C₆)alkyl substituted by one or more substituents such as lower alkyl as mentioned above, halogen as mentioned above, cyano, carboxy, protected carboxy as mentioned below, aryl which may have suitable substituent(s) as mentioned above, amidated carboxy as mentioned below. Specific examples of thus defined "ar(C₁-C₆)alkyl which may have suitable substituents"may be methylbenzyl, isobutylbenzyl, methylphenylethyl, isobutylphenylethyl, methylphe- nylpropyl, isobutylphenylpropyl, methylphenylpentyl, isobutylphenylpentyl, bis(methylphenyl)methyl, bis(propylphenyl) methyl, bis(butylphenyl)methyl, bis(isobutylphenyl)methyl, b is(chlorophenyl)methyl; (cyano)(isobutylphenyl)methyl, (carboxy)(isobutylphenyl)methyl, (benzyloxycarbonyl)(isobutylphenyl)methyl, (N,N-diethylcarbamoyl)(isobutylphenyl) methyl, (t-butylcarbamoyl)(isobutylphenyl)methyl, (phenylcarbamoyl)(isobutylphenyl)methyl, (isobutylphenylcarbamoyl)(isobutylphenyl)methyl, etc.], and the like.

Suitable "amino protective group" may be a conventional protective group, which is used in the field of organic chemistry, that is, may include acyl such as (C₁-C₆) alkanoyl (e.g. formyl, acetyl, propionyl, butyryl, isobutyryl, valeryl, isovaleryl, pivaloyl, hexanoyl, etc.), (C₁-C₆) alkoxycarbonyl (e.g. methoxycarbonyl, ethoxycarbonyl, propoxycarbonyl, butoxycarbonyl, t-butoxycarbonyl, etc.), and the like

Suitable "protected carboxy" may include an esterified carboxy group.

Suitable examples of the ester moiety of an "esterified carboxy" may be the ones such as (C₁-C₆) alkyl ester (e. g. methyl ester, ethyl ester, propyl ester, isopropyl ester, butyl ester, isobutyl ester, tert-butyl ester, pentyl ester, hexyl ester, 1-cyclopropylethyl ester, etc.) which may have at least one suitable substituent(s), for example, (C₁-C₆) alkanoy- loxy(C₁-C₆)alkyl ester (e.g. acetoxymethyl ester, propionyloxymethyl ester, butyryloxymethyl ester, valeryloxymethyl ester, pivaloyloxymethyl ester, hexanoyloxymethyl ester, 1 (or 2)-acetoxyethyl ester, 1 (or 2 or 3)-acetoxypropyl ester, 1 (or 2 or 3 or 4)-acetoxybutyl ester, 1 (or 2)-propionyloxyethyl ester, 1 (or 2 or 3)-propionyloxypropyl ester, 1 (or 2)-butyryloxyethyl ester, 1 (or 2)-isobutyryloxyethyl ester, 1 (or 2)-pivaloyloxyethyl ester, 1 (or 2)-hexanoyloxyethyl ester, isobutyryloxymethyl ester, 2-ethylbutyryloxymethyl ester, 3,3-dimethylbutyryloxymethyl ester, 1 (or 2)-pentanoyloxye- thyl ester, etc.) (C₁-C₆) alkanesulfonyl(C₁-C₆)alkyl ester (e.g. 2-mesylethyl ester, etc.), mono(or di or tri)-halo(C₁-C₆) alkyl ester (e.g. 2-iodoethyl ester, 2,2,2-trichloroethyl ester, etc.), (C₁-C₆) alkoxycarbonyloxy(lower)alkyl ester (e.g. methoxycarbonyloxymethyl ester, ethoxycarbonyloxymethyl ester, 2-methoxycarbonyloxyethyl ester, 1 -ethoxycarbonyloxyethyl ester, 1-isopropoxycarbonyloxyethyl ester, etc.), phthalidylidene(C₁-C₆)alkyl ester or (5-(C₁-Cₛ) alkyl-2-oxo-1,3-dioxol-4-yl)(C₁-C₆)alkyl ester (e.g. (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester, etc.; (C₂-C₆) alkenyl ester (e.g. vinyl ester, allyl ester, etc.); (C₂-C₆)alkynyl ester (e.g. ethynyl ester, propynyl ester, etc.); ar(C₁-C₆)alkyl ester which may have at least one suitable substituent(s) (e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester, etc.); aryl ester which may have at least one suitable substituent(s) (e.g. phenyl ester, 4-chlorophenyl ester, tolyl ester, tert-butylphenyl ester, xylyl ester, mesityl ester, cumenyl ester, etc.); phthalidyl ester; and the like.

Preferable examples of the esterified carboxy as mentioned above may include (C₁-C₆) alkoxycarbonyl (e.g. methoxycarbonyl, ethoxycarbonyl, propoxycarbonyl, isopropoxycarbonyl butoxycarbonyl, isobutoxycarbonyl, tert-butoxycarbonyl, pentyloxycarbonyl, tert-pentyloxycarbonyl, hexyloxycarbonyl, 1-cyclopropylethoxycarbonyl, etc.).

Suitable "carboxy protective group" may be the ester moiety of the above defined "protected carboxy" and may include C₁-C₆ alkyl (e.g. methyl, ethyl, etc.), ar(lower)alkyl (e.g. benzyl, etc.), and the like

Suitable "amino which may have suitable substituent(s)" is conventional one used in a pharmaceutical field and may include amino, mono or di(C₁-C₆)alkylamino (e.g. methylamino, dimethylamino, ethylamino, diethylamino, butylamino, t-butylamino, etc.), arylamino (e.g. phenylamino, etc.), C₁-C₆ alkylarylamino (e.g. isobutylphenylamino, etc.), and the like.

Suitable "heterocyclic group containing nitrogen atom" may include saturated or unsaturated monocyclic or polycyclic heterocyclic group containing at least one nitrogen atom. Especially preferable heterocyclic group may be 5- or 6- membered aliphatic heteromonocyclic group (e.g. morpholinyl, pyrrolidinyl, imidazolidinyl, piperidyl, piperazinyl, etc.), unsaturated condensed heterocyclic group such as dibenzo[6 or 7-membered unsaturated]heteromonocyclic group (e.g. phenoxazinyl, phenothiazinyl, 10,11-dihydro-5H-dibenzoazepinyl, etc.), and the like.

Suitable "amidated carboxy" may carbamoyl which may have suitable substituent(s) and may include carbamoyl, mono or di(C₁-C₆)alkylcarbamoyl (e.g. methylcarbamoyl, dimethylcarbamoyl, ethylcarbamoyl diethylcarbamoyl, butylcarbamoyl, t-butylcarbamoyl, etc.), C₁-C₆ alkylarylcarbamoyl (e.g. isobutylphenylcarbamoyl, etc. and the like.

Particularly, the preferred embodiments of R¹, R², R³, A, Q, X, Y and Z are as follows.
R¹ is carboxy;
   C₁-C₆alkoxycarbonyl, more preferably C₁-C₄alkoxycarbonyl (e.g. methoxycarbonyl, ethoxycarbonyl, etc.); or ar(C₁-C₆)alkoxycarbonyl, more preferably mono- or di- or triphenyl(C₁-C₄)alkoxycarbonyl (e.g. benzyloxycarbonyl, etc.),
R² is hydrogen;
   C₁-C₆ alkyl, more preferably C₁-C₄ alkyl (e.g. methyl, etc.)
R³ is mono- or di- or triphenyl(C₁-C₆)alkyl which may be substituted by (C₁-C₆)alkyl, or 10,11-dihydro-5H-dibenzo [b,f]azepinyl,
A is (C₁-C₆) alkylene, more preferably C₁-C₄ alkylene which may be substituted by oxo (e.g. ethylene, trimethylene, oxotrimethylene, etc.);
Q is carbonyl;
X is 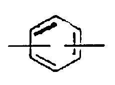
Y is bond
Z is (C₁-C₆) alkylene, more preferably C₁-C₄ alkylene (e.g. methylene, etc.);
   O ; or
   N-_{R}6
   in which
   R⁶ is hydrogen; or (C₁-C₆) alkyl, preferably C₁-C₄ alkyl (e.g. methyl, ethyl, etc.); lower alkoxycarbonyl, preferably C₁-C₄ alkoxycarbonyl (e.g. t-butoxycarbonyl, etc.);

The processes 1 to 3 for preparing the object compound (I) of the present invention are explained in detail in the following.

### Process 1

The object compound (I-a) or a salt thereof can be prepared by reacting the compound (II) or a salt thereof with the compound (II or a salt thereof.

This reaction is usually carried out in a solvent such as alcohol [e.g. methanol, ethanol, etc.], dichloromethane, benzene, N,N-dimethylformamide, tetrahydrofuran, diethyl ether or any other solvent which does not adversely affect the reaction.

The reaction may be carried out in the presence of an inorganic or an organic base such as an alkali metal hydroxide [e.g. sodium hydroxide, potassium hydroxide, etc.], an alkali metal carbonate [e.g. sodium carbonate, potassium carbonate, etc.], an alkali metal bicarbonate [e.g. sodium bicarbonate, potassium bicarbonate, etc.], alkali metal hydride (e.g. sodium hydride, potassium hydride, etc.), tri(C₁-C₆)alkylamine [e.g. trimethylamine, triethylamine, diisopropylethylamine, etc.], pyridine or its derivative [e.g. picoline, lutidine, 4-dimethylaminopyridine, etc.], or the like. In case that the base to be used is liquid, it can also be used as a solvent.

The reaction temperature is not critical, and the reaction can be carried out under cooling, at room temperature or under warming or heating.

### Process 2

The object compound (I) or a salt thereof can be prepared by reacting the compound (IV) or a salt thereof with the compound (V) or a salt thereof.

This reaction can be carried out in substantially the same manner as Process 1, and therefore the reaction mode and reaction conditions [e.g. solvents, reaction temperature, etc.] of this reaction are to be referred to those as explained in Process 1.

### Process 3

The object compound (I-e) or a salt thereof can be prepared by subjecting the compound (I-d) or a salt thereof to elimination reaction of the carboxy protective group.

In the present elimination reaction, all conventional methods used in the elimination reaction of the carboxy protective group, for example, hydrolysis, reduction, elimination using Lewis acid, etc. are applicable. When the carboxy protective group is an ester, it can be eliminated by hydrolysis or elimination using Lewis acid. The hydrolysis is preferably carried out in the presence of a base or an acid

Suitable base may include, for example, an inorganic base such as alkali metal hydroxide (e.g. sodium hydroxide, potassium hydroxide, etc.), alkaline earth metal hydroxide (e.g. magnesium hydroxide, calcium hydroxide, etc.), alkali metal carbonate (e.g. sodium carbonate, potassium carbonate, etc.), alkaline earth metal carbonate (e.g. magnesium carbonate, calcium carbonate, etc.), alkali metal bicarbonate (e.g. sodium bicarbonate, potassium bicarbonate, etc.), alkali metal acetate (e.g. sodium acetate, potassium acetate, etc.), alkaline earth metal phosphate (e.g. magnesium phosphate, calcium phosphate, etc.), alkali metal hydrogen phosphate (e.g. disodium hydrogen phosphate, dipotassium hydrogen phosphate, etc.), or the like, and an organic base such as trialkylamine (e.g. trimethylamine, triethylamine, etc.), picoline, N-methylpyrrolidine, N-methylmorpholine, 1,5-diazabicyclo[4.3.0]non-5-one, 1,4-diazabicyclo [2.2.2]octane, 1,5-diazabicyclo[5.4.0]undecene-5 -5 or the like. The hydrolysis using a base is often carried out in water or a hydrophilic organic solvent or a mixed solvent thereof.

Suitable acid may include an organic acid (e.g. formic acid, acetic acid, propionic acid, etc.) and an inorganic acid (e.g. hydrochloric acid, hydrobromic acid, sulfuric acid, etc.).

The present hydrolysis is usually carried out in an organic solvent, water or a mixed solvent thereof.

The reaction temperature is not critical, and it may suitable be selected in accordance with the kind of the carboxy protective group and the elimination method.

The elimination using Lewis acid is preferable to eliminate substituted or unsubstituted ar(C₁-Cₛ)alkyl ester and carried out by reacting the compound (Ig) or a salt thereof with Lewis acid such as boron trihalide (e.g. boron trichloride, boron trifluoride, etc.), titanium tetrahalide (e.g. titanium tetrachloride, titanium tetrabromide, etc.), tin tetrahalide (e.g. tin tetrachloride, tin tetrabromide, etc.), aluminum halide (e.g. aluminum chloride, aluminum bromide, etc.), trihaloacetic acid (e.g. trichloroacetic acid, trifluoroacetic acid, etc.) or the like. This elimination reaction is preferably carried out in the presence of cation trapping agents (e.g. anisole, phenol, etc.) and is usually carried out in a solvent such as nitroalkane (e.g. nitromethane, nitroethane, etc.), alkylene halide (e.g. methylene chloride, ethylene chloride, etc.), diethyl ether, carbon disulfide or any other solvent which does not adversely affect the reaction. These solvents may be used as a mixture thereof.

The reduction elimination can be applied preferably for elimination of the protective group such as halo(C₁-Cₛ) alkyl (e.g. 2-iodoethyl, 2,2,2-trichloroelhyl, etc.) ester, ar(lower)alkyl (e.g. benzyl, etc.) ester or the like.

The reduction method applicable for the elimination reaction may include, for example, reduction by using a combination of a metal (e.g. zinc, zinc amalgam, etc.) or a salt of chromium compound (e.g. chromous chloride, chromous acetate, etc.) and an organic or an inorganic acid (e.g. acetic acid, propionic acid, hydrochloric acid, etc.); and conventional catalytic reduction in the pressure of a conventional metallic catalyst (e.g. palladium carbon, Raney nickel, etc.).

The reaction temperature is not critical, and the reaction is usually carried out under cooling, at ambient temperature or under warming.

The object compound (I) of the present invention can be isolated and purified in a conventional manner, for example, extraction, precipitation, fractional crystallization, recrystallization, chromatography and the like.

The object compound (I) thus obtained can be converted to its salt by a conventional method.

The object compound (I) of the present invention is useful as a testosteron 5a-reductase inhibitor and effective to testosteron 5a-reductase mediated diseases such as prostatism, prostatic hypertrophy, prostatic cancer, alopecia, hirsutism (e.g. female hirsutism, etc.), androgenic alopecia (or male-pattern baldness), acne (e.g. acne vulgaris, pimple etc.), other hyperandrogenism, and the like.

In order to illustrate the usefulness of the object compounds (I), pharmacological activity of representative compounds of the present invention is shown below.

### [1 ] Test Compound :

(1) 4-[3-[3-[Bis(4-isobutylphenyl)methylamino]benzoyl]indol-1-yl]butyric acid
(2) 4-[3-[3-[Bis(4-isobutylphenyl)methylamino]benzoyl]-2-methylindol-1 -yl]butyrlc acid
(3) 4-[3-[4-[Bis(4-isobutylphenyl)methoxy]benzoyl]indol-1 -yl]butyric acid
(4) 4-[3-[4-[1-(4-Isobutylphenyl)ethoxy]benzoyl]indol-1-yl]butyric acid
(5) 4-[3-[3-[2,2-Bis(4-isobutylphenyl)ethyl]benzoyl]indol-1 -yl]butyric acid
(6) 4-[3-[3-(10,11 -Dihydro-5H-dibenz[b,f]azepin-5-ylmethyl)benzoyl]indol-1 -yl]butyric acid

### [2] Inhibitory activity on testosterone 5a-reductase in rats :

### Test Methods

### i) Materials

### 1,2,6,7-³H-Testosterone (85-105 Ci/mmol):

1,2,6,7-³H-Testosterone (85-105 Ci/mmol) is a mixture of 1,2,6,7-³H-testosterone and testosterone which includes 85-105 Ci of 1,2,6,7-³H-testosterone per mmol of testosterone and is purchased from New England Nuclear, Boston, Mass., U.S.A..

Aquazol-2 (Aquazol-2 Universal LSC Cocktail) :
trademark, purchased from New England Nuclear, Boston, Mass., U.S.A.

### ii) Preparation of prostatic testosterone 5a-reductase

Mature Spraque-Dawley male rats (7-8 weeks old) were sacrificed by diethyl ether. The ventral prostates were dissected to be free of their capsules and their combined volume was measured by displacement in several milliliters of ice-cold medium A (0.32 M sucrose, 0.1 mM dithiothreitol and 20 mM sodium phosphate, pH 6.5). Unless specified, all the following procedures were carried out at 0-4°C. The prostates were drained, minced, and then homogenized in 3-4 tissue volumes of medium A with Pyrex-glass homogenizer. The homogenate was fractioned by differential centrifugations at 3,000 g for 15 minutes. The resulting pellets were resuspended in medium A. The suspension (20-30 mg protein/ml) was stored at -80°C

### iii) Testosterone 5a-reductase assay

The reaction solution contains 1 mM dithiothreitol, 40 mM sodium phosphate pH 6.5, 50 µM NADPH, 1,2,6,7-³H-testosterone/testosterone (2.2 x 10-⁹ M) and the suspension prepared above (0.8 mg of protein) in a total volume of 565 µl. Test Compound was added in 10 µl of 10% ethanol whereas control tubes received the same volume of 10% ethanol. The reaction was started with the addition of the enzyme suspension. After incubation at 37°C for 30 minutes, the reaction was extracted with 1 ml of ethyl acetate Fifty µl of ethyl acetate phase was chromatographed on a Merck silica plastic sheet Kieselgel 60 F₂₅₄, using ethyl acetate :
cyclohexane (1:1) as the developing solvent system. The plastic sheet was air dried and cut the testosterone and the 5a-dihydrotestosterone areas. The radioactivity was counted in 5 ml of Aquazol-2 in Packard scintillation counter (PACKARD TRI - CARB 4530), and an inhibitory ratio was calculated.

### [3] Test Results :

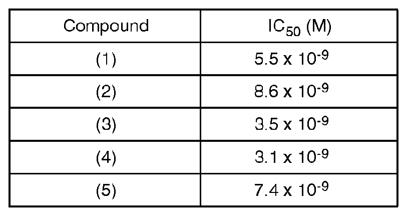

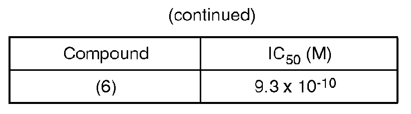

For therapeutic or preventive administration, the object compound (I) of the present invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration The pharmaceutical preparation may be in solid form such as tablet, granule, powder, capsule, or liquid form such as solution, suspension, syrup, emulsion, lemonade, lotion and the like.

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases or conditions, a kind of the compound (I) to be applied, etc. In general amounts between 0.01 mg and about 500 mg or even more per day may be administered to a patient. An average single dose of about 0.05 mg 0.1 mg, 0.25 mg, 0.5 mg, 1 mg, 20 mg, 50 mg, 100 mg of the object compound (I) of the present invention may be used in treating diseases.

The following Preparations and Example are given for the purpose of illustrating the present invention.

The following Preparations and Examples are given for the purpose of illustrating the present invention.

### Preparation 1

A solution of 3-nitrobenzoyl chloride (4.76 g) in dichloromethane (20 ml) was added to a suspension of aluminum chloride (3.42 g) in dichloromethane (50 ml) at 25°C, and the mixture was stirred at the same temperature for an hour. A solution of indole (3.0 g) in dichloromethane (20 ml) was added to the mixture at 25°C. After stirring for an hour at 25°C, the reaction mixture was poured into a mixture of ethyl acetate and ice water. The organic layer was separated, washed with water, and dried over magnesium sulfate. After evaporation of solvent, the crystalline residue was recrystallized from ethyl acetate to give 3-(3-nitrobenzoyl)indole (2.37 g) as pale red crystals. The mother liquid was purified by column chromatography on silica gel (20 g) with chloroform as eluent to give another crystals of 3-(3-nitrobenzoyl) indole (0.277 g).
NMR (CDCl₃-CD₃0D, δ) : 7.21-7.35 (2H, m), 7.42-7.55 (1 H, m), 7.68-7.79 (2H, m), 8.13 (1 H, dif-dd, J=7.5Hz), 8.24-8.35 (1 H, m), 840 (1 H, dif-dd, J=7.5Hz), 8.60 (1 H, dif-d)

### Preparation 2

A mixture of 3-(3-nitrobenzoyl)indole (2.09 g), ethyl 4-bromobutyrate (1.614 g) and potassium carbonate (3.118 g) in N,N-dimethylformamide (20 ml) was stirred at 25°C overnight. The reaction mixture was poured into a mixture of ethyl acetate and 1 N hydrochloric acid. The organic layer was separated, washed with water and brine, and dried over magnesium sulfate. After evaporation of solvent, the crystalline residue was recrystallized from a mixture of ethyl acetate and hexane to give ethyl 4-[3-(3-nitrobenzoyl)indol-1-yl]butyrate (2.71 g) as colorless crystals.
NMR (CDC1₃, 8): 1.20 (3H, t, J=7.5Hz), 2.12-2.40 (4H, m), 4.10 (2H, q, J=7.5Hz), 4.30 (2H, t, J=7.5Hz), 7.30-7.50 (3H, m), 7.58 (1 H, s), 7.70 (1 H, t, J=8Hz), 8.27 (1 H, dif-dd, J=7.5Hz), 8.35-8.48 (2H, m), 8.68 (1 H, dif-d)

### Preparation 3

A mixture of ethyl 4-[3-(3-nitrobenzoyl)indol-1-yl]butyrate (1.60 g), 1N aqueous sodium hydroxide (11 ml) and 1,4-dioxane (50 ml) was stirred at 25°C for 14 hours. After evaporation of the organic solvent, 1N hydrochloric acid (20 ml) was added to the aqueous solution and the mixture was extracted with ethyl acetate. The extract was washed with water, dried over magnesium sulfate, and evaporated in vacuo. The crystalline residue was recrystallized from a mixture of ethyl acetate and hexane to give 4-[3-(3-nitrobenzoyl)indol-1-yl]butyric acid (1.28 g) as colorless crystals.
NMR (CDCl₃-CD₃0D, δ) : 2.10 (2H, m), 2.35 (2H, t, J=7.5Hz), 4.30 (2H, t, J=7.5Hz), 7.30-7.55 (3H, m), 7.60 (1 H, s), 7.72 (1 H, t, J=₇.₅Hz), 8.16 (1 H, dif-dd, J=₇.₅Hz), 8.31-8.48 (2H, m), 8.65 (1 H, dif-d)

### Preparation 4

A mixture of 4-[3-(3-nitrobenzoyl)indol-1-yl]butyric acid (1.20 g), 10% palladium on carbon (300 mg), methanol (12 ml) and 1,4-dioxane (12 ml) was stirred under hydrogen atmosphere (3 atm) at 25°C for 45 minutes. The mixture was filtered and the filtrate was evaporated to give 4-[3-(3-aminobenzoyl)indol-1-yl]butyric acid (982 mg) as yellow oil.
NMR (CDCl₃-CH₃0D, δ) : 2.15-2.45 (4H, m); 4.32 (2H, t, J-7.5Hz), 6.97 (1 H, m), 7.15-7.60 (6H, m), 7.72 (1 H, s), 8.45 (1 H, m)

### Preparation 5

The following compound was obtained according to a similar manner to that of Preparation 1. 3-(4-Nitrobenzoyl)indole
NMR (CDCl₃-CD₃0D, δ) : 7.2-7.45 (2H, m), 7.5-7.6 (2H, m), 7.72 (2H, d, J=7.5Hz), 8.2-8.3 (1 H, m), 8.31 (2H, d, J=7.5Hz)

### Preparation 6

The following compound was obtained according to a similar manner to that of Preparation 2. Ethyl 4-[3-(4-nitrobenzoyl)indol-1-yl]butyrate
NMR (CDCl₃, δ) : 1.20 (3H, t, J=7.5Hz), 2.2-2.4 (4H, m), 4.10 (2H, q, J=7.5Hz), 4.27 (2H, t, J=7.5Hz), 7.35-7.5 (3H, m), 7.52 (1 H, s), 7.95 (2H, d, J=8Hz), 8.35 (2H, d, J=8Hz), 8.4-8.5 (1 H, m)

### Preparation 7

The following compound was obtained according to a similar manner to that of Preparation 3. 4-[3-(4-Nitrobenzoyl)indol-1-yl]butyric acid
NMR (CDCl₃-CD₃0D, δ) : 1.8-2.0 (2H, m), 2.15 (2H, t, J=7.5Hz), 4.12 (2H, t, J=₇.₅Hz), 7.1-7.25 (2H, m), 7.45-7.55 (1H m), 7.81 (1 H, s), 7.85 (2H, d, J=8Hz), 8.10-8.15 (1 H, m), 8.18 (2H, d, J=8Hz)

### Preparation 8

The following compound was obtained according to a similar manner to that of Preparation 4. 4-[3-(4-Aminobenzoyl)indol-1-yl]butyric acid
NMR (CDCl₃-CD₃0D, δ) : 2.20 (2H, quintet, J=7.5Hz), 2.33 (2H, t, J=₇.₅Hz), 4.36 (2H, t, J=₇.₅Hz), 6.75 (2H, d, J=8Hz), 7.20-7.40 (2H, m), 7.50 (1 H, dd, J=2, 8Hz), 7.65-7.80 (1 H, m), 7.70 (2H, d, J=8Hz), 8.25 (1 H, dd; J=2, 8Hz)

### Preparation 9

To a suspension of isopropyltriphenylphosphonium iodide (24.7 g) in tetrahydrofuran (100 ml) was added a solution of potassium tert-butoxide (7.71 g) in tetrahydrofuran (50 ml) at 25°C over 20 minutes, and the mixture was stirred for 30 minutes at the same temperature. A solution of 3-cyanobenzaldehyde (5.0 g) in tetrahydrofuran (50 ml) was added at 0°C over 20 minutes, and the mixture was stirred at 25°C for 1 hour. The mixture was poured into a mixture of ethyl acetate and 1N hydrochloric acid. The organic layer was separated, washed with water and brine, and dried over magnesium sulfate. The residue was chromatographed on silica gel (200 g) eluting with 10% ethyl acetate in hexane to give 3-(2-methyl-1-propenyl)benzonitrile (4.21 g) as a pale yellow oil.
NMR (CDCl₃, δ) : 1.83 (3H, d, J=1.5Hz), 1.91 (3H, d, J=1.5Hz), 6.22 (1 H, s), 7.31-7.56 (4H, m)

### Preparation 10

The following compound was obtained according to a similar manner to that of Preparation 9. 1-(2-Methyl-1-propenyl)-3-nitrobenzene
NMR (CDCl₃, δ) : 1.88 (3H, d, J=1.5Hz), 1.93 (3H, d, J=1.5Hz), 6.30 (1 H, s), 7.49-7.60 (2H, m), 7.95-8.13 (2H, m)

### Preparation 11

To a solution of 3-(2-methyl-1-propenyl)benzonitrile (5.80 g) in methanol (50 ml) was added 10% palladium on activated carbon (1 g), and the mixture was stirred under hydrogen atmosphere (3 atm) at 25°C for 1 hour. The catalyst was filtered off and the filtrate was evaporated to give 3-isobutylbenzonitrile (5.52 g) as a pale yellow oil.
NMR (CDCl₃, 8): 0.90 (6H, d, J=7.5Hz), 1.70-1.98 (1 H, m), 2.50 (2H, d, J=7.5Hz), 7.31-7.60 (4H, m)

### Preparation 12

To a solution of 3-isobutylbenzonitrile (7.0 g) in toluene (150 ml) was added a 1.5M solution of diisobutylaluminum hydride in toluene (88 ml) at 25°C over 30 minutes and the mixture was stirred at 25°C for 2 hours. Another 1.5M solution of diisobutylaluminum hydride in toluene (30 ml) was added, and the mixture was stirred at 25°C for 1 hour. The mixture was poured into a mixture of ether, aqueous ammonium chloride and 1 N hydrochloric acid. The organic layer was separated, washed with water and brine, and dried over magnesium sulfate. After evaporation of the solvent, the residue was chromatographed on silica gel (150 g) eluting with a mixture of toluene and hexane (2:1) to give 3-isobutylbenzaldehyde (4.72 g) as a colorless oil.
NMR (CDCl₃, 8): 0.90 (6H, d, J-7.5Hz), 1.80-2.05 (1 H, m), 2.58 (2H, d, J-7.5Hz), 7.35-7.50 (2H, m), 7.60-7.79 (2H, m), 10.00 (1 H, s)

### Preparation 13

To a solution of 1-(2-methyl-1-propenyl)-3-nitrobenzene (4.57 g) in a mixture of methanol (50 ml) and 6N hydrochloric acid (14 ml) was added 10% palladium on activated carbon (1 g), and the mixture was stirred under hydrogen atmosphere (3 atm) at 25°C for 3 hours. The catalyst was filtered off, and the filtrate was evaporated. The residue was poured into a mixture of ethyl acetate and aqueous sodium bicarbonate. The organic layer was separated, washed with water and brine, and dried over magnesium sulfate. Evaporation of the solvent gave 3-isobutylphenylamine (3.98 g) as a brown oil.
NMR (CDCl₃-CD₃0D, δ) : 0.93 (6H, d, J=7.5Hz), 1.75-2.05 (1H, m), 2.52 (2H, d, J=7.5Hz); 7.13-7.32 (3H, m), 7.32-7.55 (1 H, m)

### Preparation 14

To a solution of 3-isobutylphenylamine (3.80 g) in 48% hydrobromic acid (10 ml) was added aqueous sodium nitrite (2.11 g, 2 ml) below 10°C over 20 minutes. The reaction mixture was added to a solution of cuprous bromide (7.3 g) in 48% hydrobromic acid (5 ml) at 25°C. After stirred at 25°C for 1 hour, the mixture was extracted with hexane two times. The extracts were combined, washed with water and brine, and dried over magnesium sulfate. After evaporation of the solvent, the residue was chromatographed on silica gel (150 g) eluting with hexane to give 1-bromo-3-isobutylbenzene (2.61 g) as a colorless oil.
NMR (CDCl₃, δ) : 0.92 (6H, d, J=7.5Hz), 1.72-1.98 (1 H, m), 2.43 (2H, d, J=7.5Hz), 7.00-7.22 (2H; m), 7.22-7.43 (2H, m)

### Preparation 15

A mixture of 1-bromo-3-isobutylbenzene (3.16 g), magnesium (1.08 g), 1,2-dibromoethane (2.78 g) and iodine (10 mg) in tetrahydrofuran (10 ml) was refluxed for 1.5 hours. The mixture was cooled to 25°C, and a solution of 3-isobutylbenzaldehyde (2.40 g) in tetrahydrofuran (10 ml) was added at 25°C. After stirred for 1 hour at the same temperature, the mixture was poured into a mixture of ethyl acetate and 1 N hydrochloric acid. The organic layer was separated, washed with water and brine, and dried over magnesium sulfate. After evaporation of the solvent, the residue was chromatographed on silica gel (200 g) eluting with 5% ethyl acetate in hexane to give bis(3-isobutylphenyl)methanol (2.90 g) as a colorless oil.
NMR (CDCl₃, δ) : 0.89 (12H, d, J=7.5Hz), 1.70-1.95 (2H, m), 2.46 (4H, d, J=7.5Hz), 5.80 (1 H, s), 6.97-7.09 (2H, m), 7.09-7.30 (6H, m)

### Preparation 16

A mixture of bis(3-isobutylphenyl)methanol (2.87 g) and oxalyl chloride (1 ml) in dichloromethane (10 ml) was stirred at 25°C for 2 hours. After evaporation of the solvent, the oily residue was distilled to give bis(3-isobutylphenyl) chloromethane (2.45 g) as pale yellow oil.
bp : 150°C (0.7 mmHg)
NMR (CDCl₃, δ) : 0.89 (12H, d, J=7.5Hz), 1.70-1.95 (2H, m), 2.45 (4H, d, J=7.5Hz), 6.10 (1 H, s), 7.02-7.14 (2H, m), 7.16-7.30 (6H, m)

### Preparation 17

Benzyl 3-chloroformylpropionate was prepared from benzyl hydrogen succinate (1.96 g) and oxalyl chloride (0.9 ml) in an usual manner. A solution of sodium phenolate, which was prepared from phenol (1.77 g) and sodium hydride (60% dispersion in mineral oil, 1.13 g) in tetrahydrofuran (20 ml), was added to a solution of the acid chloride in tetrahydrofuran (20 ml) at 0°C. After stirred at 0°C for 30 minutes, the mixture was poured into a mixture of ethyl acetate and 1 N hydrochloric acid. The organic layer was separated, washed with water, and dried over magnesium sulfate. After evaporation of the solvent, the residue was chromatographed on silica gel (50 g) eluting with a mixture of ethyl acetate and hexane (1:10 → 1:5) to give benzyl phenyl succinate (2.15 g) as colorless crystals.
NMR (CDCl₃, 8): 2.64-2.96 (4H, m), 5.17 (2H, s), 7.05 (2H, m), 7.2-7.45 (8H, m)

### Preparation 18

To a solution of 4-[2,2-bis(4-isobutylphenyl)ethyl]benzoic acid (400 mg) in dichloromethane (10 ml) were added oxalyl chloride (0.1 ml) and a drop of N,N-dimethylformamide at 0°C. After stirred at 20°C for 1 hour, the mixture was evaporated to give 4-[2,2-bis(4-isobutylphenyl)ethyl]benzoyl chloride (450 mg) as pale yellow oil.
NMR (CDCl₃, δ) : 0.88 (12H, d, J=7.5Hz), 1.70-1.95 (2H, m), 2.41 (4H, d, J=7.5Hz), 3.40 (2H, d, J=7.5Hz), 4.16 (1 H, t, J-7.5Hz), 6.95-7.18 (10H, m), 7.90 (2H; d, J=8Hz)

### Preparation 19

To a suspension of powdered potassium hydroxide (168 g) in toluene (300 ml) was added 4'-isobutylacetophenone (52.9 g), 18-crown-6 (79 mg) and methyliodide (149 ml). The mixture was stirred at 70°C for 2 hours. The precipitate was filtered off and the filtrate was evaporated. The residue oil was distillated under reduced pressure to give 2,2-dimethyl-4'-isobutylpropiophenone as a colourless oil (23.0 g).
bp: 100-105°C (0.2 mmHg)
NMR (CDCl₃, δ) : 0.90 (6H, d, J=7Hz), 1.36 (9H, s), 1.90 (1 H, m), 2.50 (2H, d, J=7Hz), 7.17 (2H, d, J=8Hz), 7.68 (2H, d, J=8Hz)

### Preparation 20

Propionyl chloride (13.0 ml) was added to a suspension of aluminum chloride (20.0 g) in dichloromethane (200 ml) at 0°C. After the mixture was stirred at 0°C for 1 hour, isobutylbenzene (23.6 ml) was added to the mixture. The mixture was stirred at 0°C for 2 hours and poured into ice water. The organic layer was washed with water, dried over magnesium sulfate and evaporated. The residual oil was distilled under reduced pressure to give 4'-isobutylpropiophe- none as a colourless oil (24.4 g).
NMR (CDCl₃, δ) : 0.92 (6H, d, J=7Hz), 1.21 (3H, t, J=7Hz), 1.90 (1 H, m), 2.53 (2H, d, J=7Hz), 3.00 (2H, q, J=₇Hz), 7.23 (2H, d, J=8Hz), 7.90 (2H, d, J=8Hz)

### Preparation 21

Sodium borohydride (4.72 g) was added to a solution of 2,2-dimethyl-4'-isobutylpropiophenone (22.7 g) in isopropyl alcohol (150 ml). The mixture was stirred at 50°C for 2 hours and poured into ice water. After acidified with 6N-hydrochloric acid, the mixture was extracted with ethyl acetate. The organic layer was washed with water dried over magnesium sulfate and evaporated to give 2,2-dimethyl-1-(4-isobutylphenyl)propanol as a colourless oil (21.6 g).
NMR (CDCl₃, δ) : 0.90 (6H, d, J=7Hz), 0.92 (9H, s), 1.85 (1 H, m), 2.45 (2H, d, J=7Hz), 4.38 (1 H, s), 7.08 (2H, d, J=8Hz), 7.23 (2H; d, J=8Hz)

### Preparation 22

The following compound was obtained according to a similar manner to that of Preparation 21. 1-(4-Isobutylphenyl)propanol as a colourless oil
NMR (CDCl₃, δ) : 0.88 (6H, d, J=7Hz), 0.89 (3H; t, J=7Hz), 1.6-2.0 (3H, m), 2.47 (2H, d, J=7Hz), 4.57 (1 H, t, J=₇Hz), 7.13 (2H, d, J=8Hz), 7.25 (2H, d, J=8Hz)

### Preparation 23

To a mixture of 2,2-dimethyl-1-(4-isobutylphenyl)propanol (22.8 g) and carbon tetrabromide (61.8 g) in tetrahydrofuran (250 ml) was added triphenylphosphine (48.9 g) under nitrogen atmosphere at 0°C. The mixture was stirred at room temperature for 6 hours. After the white solid was filtered off, the filtrate was evaporated. n-Hexane (250 ml) was added to the residue and the precipitate was filtered off. The filtrate was evaporated and the residual oil was distilled under reduced pressure to give 1-(1-bromoneopentyl)-4-isobutylbenzene as a colourless oil (10.3 g).
bp: 120-125°C (0.2 mmHg)
NMR (CDCl₃, δ) : 0.90 (6H, d, J=7Hz), 1.05 (9H, s), 1.86 (1 H, m), 2.45 (2H, d, J=7Hz), 4.85 (1 H, s), 7.05 (2H, d, J=8Hz), 7.28 (2H; d, J=8Hz)

### Preparation 24

The following compounds were obtained according to a similar manner to that of Preparation 23.
(1) 1-(1-Bromopropyl)-4-isobutylbenzene
   NMR (CDCI₃, δ) : 0.90 (6H; d, J=7Hz), 1.01 (3H, t, J=7Hz), 1.7-2.0 (1 H, m); 2.0-2.4 (2H, m), 2.45 (2H, d, J=7Hz), 4.89 (1 H, t, J=7Hz), 7.11 (2H, d, J=8Hz), 7.30 (2H, d, J=8Hz),
(2) 2-Bromo-2-(4-isobutylphenyl)acetonitrile
   NMR (CDCI₃, δ) : 0.90 (6H, d, J=7Hz), 1.89 (1 H, m), 2.51 (2H, d, J=7Hz); 5.50 (1 H, s), 7.21 (2H, d, J=9Hz), 7.47 (2H, d, J=9Hz)
(3) Benzyl 2-bromo-2-(4-isobutylphenyl)acetate
   NMR (CDCI₃, δ) : 0.90 (6H, d, J=7Hz), 1.85 (1 H, m), 2.48 (2H, d; J=7Hz), 5.20 (2H, dd; J=11 Hz), 5.38 (1 H, s), 7.12 (2H, d, J=9Hz), 7.31 (5H, s), 7.43 (2H, d, J=9Hz)

### Preparation 25

A solution of methylmagnesium bromide in ether (4 ml) was added to a solution of 3-isobutylbenzaldehyde (1.0 g) in tetrahydrofuran (10 ml) at 0°C. After stirred at 0°C for 30 minutes, the mixture was partitioned between ether and 1N hydrochloric acid. The organic layer was separated, washed with water and brine, and dried over magnesium sulfate. After evaporation of the solvent, the residue was chromatographed on silica gel (30 g) eluting with a mixture of ethyl acetate and hexane (1:10) to give 1-(3-isobutylphenyl)ethanol (1.12 g) as an oil.
NMR (CDCl₃, δ) : 1.50 (3H, d, J=8Hz), 1.90 (6H, d, J=8Hz), 1.78-2.00 (1 H, m), 2.48 (2H, d, J=8Hz), 4.87 (1 H, q, J=8Hz), 7.00-7.32 (4H, m)

### Preparation 26

A mixture of 1-(3-isobutylphenyl)ethanol (1.1 g), triphenylphosphine (3.24 g) and carbon tetrabromide (4.09 g) in ether (20 ml) was stirred at 25°C for 1 hour. The precipitates were filtered off, and the filtrate was evaporated to give 1-(1-bromoethyl)-3-isobutylbenzene (1.49 g) as an oil.
NMR (CDCl₃, δ) : 1.90 (6H, d, J=8Hz), 1.75-1.98 (1 H, m), 2.04 (3H, d, J=8Hz). 2.48 (2H, d, J=8Hz), 5.20 (1 H, q, J=8Hz), 7.00-7.10 (1 H, m), 7.18-7.30 (2H, m), 7.40-7.75 (1 H, m)

### Preparation 27

A mixture of methyl 3-hydroxybenzoate (939 mg), 1-(1-bromoethyl)-3-isobutylbenzene (1.49 g) and potassium carbonate (1.71 g) in N,N-dimethylformamide (20 ml) was stirred at 25°C overnight. The mixture was partitioned between ether and 1 N hydrochloric acid. The organic layer was separated, washed with water and brine, dried over magnesium sulfate, and evaporated. The residue was chromatographed on silica gel (30 g) eluting with a mixture of ethyl acetate and hexane (1:10) to give methyl 3-[1-(3-isobutylphenyl)ethyl]benzoate (1.45 g) as an oil.
NMR (CDCl₃, δ) : 0.85 (3H, d, J=8Hz), 0.87 (3H, d, J=8Hz), 1.65 (3H, d, J=8Hz), 1.70-1.95 (1 H, m), 2.46 (2H, d, J=8Hz), 3.87 (3H, s), 5.34 (1 H, q, J=8Hz), 6.98-7.10 (2H, m), 7.13-7.30 (4H, m), 7.52-7.58 (2H, m)

### Preparation 28

1 N aqueous solution of sodium hydroxide (6 ml) was added to a solution of methyl 3-[1-(3-isobutylphenyl)ethoxy] benzoate(1.40 g) in a mixture of 1.4-dioxane (12 ml) and methanol (6 ml). The reaction mixture was stirred at 25°C for 18 hours, and then poured into a mixture of ether and 1 N hydrochloric acid. The organic layer was separated, washed with water and brine, dried over magnesium sulfate, and evaporated. The crystalline residue was washed with hexane to give 3-[1-(3-isobutylphenyl)ethoxy]benzoic acid (1.20 g) as colorless crystals.
NMR (CDCl₃, δ) : 0.84 (3H, d, J=8Hz), 0.87 (3H, d, J=8Hz), 1.67 (3H, d, J=8Hz), 1.72-1 95 (1 H, m), 2.46 (2H, d, J=8Hz), 5.25 (1 H, q, J=8Hz), 7.00-7.34 (6H, m), 7.58-7.66 (2H, m)

### Preparation 29

A mixture of indole (11.7 g), ethyl 4-bromobutyrate (58.5 g) and potassium carbonate (41.5 g) in N;N-dimethylformamide (500 ml) was heated at 50°C for 10 hours. The mixture was filtered and the filtrate was poured into a mixture of ethyl acetate and ice water. The organic layer was separated, washed with water, and dried over magnesium sulfate. After evaporation of the solvent, the residue was chromatographed on silica gel (1 kg) eluting with a mixture of methanol, chloroform and hexane (1:50:50) to give ethyl 4-(1-indolyl)butyrate (2.86 g) as a blue oil.
NMR (CDCl₃, δ) : 1.24 (3H, t, J=7Hz), 2.0-2.4 (4H, m), 4.13 (2H, q, J=7Hz), 4.22 (2H, t, J=7Hz), 6.50 (1H, d, J=3Hz), 7.0-7.3 (3H, m), 7.36 (1 H, d, J=₇.₅Hz), 7.63 (1 H, d, J=₇.₅Hz)

### Preparation 30

The following compounds were obtained according to a similar manner to that of Preparation 1.
(1) 5-Chloro-3-(3-nitrobenzoyl)indole
   mp : 265-266°C
   NMR (DMSO-d₆, δ) : 7.32 (1 H, dd, J=2.5, 8Hz), 7.57 (1 H, d, J=8Hz), 7.86 (1 H, t, J=8Hz), 8.17-8.29 (3H, m), 8.41-8 50 (2H, m)
(2) 2-Methyl-3-(3-nitrobenzoyl)indole
   mp: 219-221 °C (dec.)
   NMR (CDCl₃-CD₃0D, δ) : 2.48 (3H, s), 6.88-7.18 (3H, m), 7.29 (1 H, d, J=8Hz), 7.60 (1 H, t, J=8Hz), 7.95 (1 H, dif-dd, J=8Hz), 8.31 (1 H, dif-dd, J=8Hz), 8.46 (1 H, dif-d)
(3) Ethyl 4-[3-[3-(chloromethyl)benzoyl]indol-1-yl]butyrate
   mp : 87-88°C
   NMR (CDCI₃, 8): 1.21 (3H, t; J=7Hz), 2.1-2.4 (4H, m), 4.11 (2H, q, J-7Hz), 4.26 (2H, t, J=7Hz), 4.66 (2H, s), 7.3-7.7 (6H, m), 7.78 (1 H, d, J=7.5Hz), 7.86 (1 H, broad s), 8.4-8.5 (1 H, m)
(4) 3-(3-Methoxybenzoyl)indole
   NMR (DMSO-d₆, δ) : 4.52 (3H, s), 6.92-7.02 (1 H, m), 7.12-7.40 (6H, m); 7.63 (1 H, s), 8.15-8.22 (1 H, m)
(5) 3-(4-Methoxybenzoyl)indole
   mp : 203-205°C
   NMR (DMSO-d₆, δ) : 3.70 (3H, s), 6.93 (2H, d, J=8Hz), 7.00-7.19 (2H, m), 7.30-7.44 (1 H, m), 7.67 (2H, d), 7.31 (1 H, s), 8.02-8.15 (1 H, m)

### Preparation 31

3-[2,2-Bis(4-isobutylphenyl)ethyl]benzoic acid (1 g) was converted to 3-[2,2-bis (4-isobutylphenyl)ethyl]benzoyl chloride (1.13 g) with oxalyl chloride as an usual manner. To a solution of indole (0.918 g) in tetrahydrofuran (15 ml) was added 3M solution of methyl magnesium bromide in ether (3 ml) at 20°C, and the mixture was stirred at 20°C for 1 hour. A solution of 3-[2,2-bis(4-isobutylphenyl)ethyl]benzoyl chloride (1.13 g) in tetrahydrofuran (15 ml) was added to the mixture at 20°C. After stirred at 20°C for 1 hour, the mixture was poured into a mixture of 1 N-hydrochloric acid and ethyl acetate. The organic layer was separated, washed with water; aqueous sodium bicarbonate and brine, and dried over magnesium sulfate. After evaporation of the solvent, the residue was purified by column chromatography on silica gel (50 g) eluting with a mixture of hexane and ethyl acetate (20:1) to give 3-[3-[2,2-bis(4-isobutylphenyl)ethyl] benzoyl]indole (468 mg) as pale red crystals.
mp: 72-76°C
NMR (CDCl₃, δ) : 0.88 (12H, d, J=7.5Hz), 1.67-1.92 (2H, m), 2.41 (4H, d, J=7.5Hz), 3.39 (2H, d, J=7.5Hz), 4.20 (1 H, t, J=₇.₅Hz), 6.93-7.47 (14H, m), 7.61 (1H, dif-dd, J=8Hz), 8.35-8.47 (1H, m), 8.57 (1H, broad s)

### Preparation 32

To a solution of indole (366 mg) in tetrahydrofuran (5 ml) was added 3M solution of methyl magnesium bromide in ether (1.5 ml) at 20°C, and the mixture was stirred at 20°C for 1 hour. A solution of 4-[2,2-bis(4-isobutylphenyl)ethyl] benzoyl chloride (450 mg) in tetrahydrofuran (5 ml) was added to the mixture at 20°C. After stirred at 20°C for 30 minutes, the mixture was poured into a mixture of 1N hydrochloric acid and ethyl acetate. The organic layer was separated, washed with water, aqueous sodium bicarbonate and brine, and dried over magnesium sulfate. After evaporation of the solvent, the residue was purified by column chromatography on silica gel (10 g) eluting with a mixture of hexane and ethyl acetate (20:1) to give 3-[4-[2,2-bis(4-isobutylphenyl)ethyl]benzoyl]indole (90 mg) as colorless crystals.
mp: 157-160°C
NMR (CDCl₃, 8): 0.87 (12H, d, J-7.5Hz), 1.72-1.95 (2H, m), 2.42 (2H, d, J-7.5Hz), 3.41 (2H, d, J-7.5Hz), 4.19 (1 H, t, J=7.5Hz), 6.94-7.20 (11 H, m), 7.25-7.46 (1 H, m), 7.46-7.50 (1 H, m), 7.59-7.72 (3H, m), 8.30-8.44 (1 H, m), 8.61 (1 H, s)

### Preparation 33

A mixture of oxalyl chloride (562 mg) and N,N-dimethylformamide (0.57 ml) in dichloromethane (10 ml) was stirred at 25°C for 1 hour. The mixture was cooled to -40°C, and a solution of 3-[1-(3-isobutylphenyl)ethoxy]benzoic acid (1.1 g) in dichloromethane (5 ml) was added to the mixture. The reaction mixture was stirred at -40°C for 1 hour, and then was partitioned between hexane and ice-water. The organic layer was separated, washed with water and brine, dried over magnesium sulfate, and evaporated to give the acid chloride. A solution of methylmagnesium bromide in ether (3M, 3.1 ml) was added to a solution of indole (1.94 g) in tetrahydrofuran (20 ml) at 25°C. The reaction mixture was stirred at 25°C for 1 hour, and then a solution of the acid chloride in tetrahydrofuran (10 ml) was added at the same temperature. After stirred at 25°C for 1 hour, the mixture was poured into a mixture of ethyl acetate and 1 N hydrochloric acid The organic layer was separated, washed with water and brine, dried over magnesium sulfate, and evaporated. The residue was chromatographed on silica gel (30 g) eluting with a mixture of ethyl acetate and hexane (10:1-4:1) to give 3-[3-[1-(3-isobutylphenyl)ethoxy]benzoyl]indole (650 mg) as an oil.
NMR (CDCl₃, δ) : 0.75-0.80 (6H, m), 1.60-1.90 (4H, m), 2.42 (2H, d, J=8Hz), 5.32 (1 H, q, J=8Hz), 6.95-7.45 (12H, m), 8.16-8.30 (1H, m)

### Preparation 34

The following compounds were obtained according to a similar manner to that of Preparation 33.
(1) 3-[3-(10,11-Dihydro-5H-dibenz[b,f]azepin-5-ylmethyl)benzoyl]indole
   NMR (DMSO-d₆, δ) : 3.16 (4H, s), 5.06 (2H, s), 6.92 (2H, t, J=8Hz), 7.05-7.30 (8H, m), 7.40-7.60 (4H, m), 7.65 (1 H, dd, J=1 , 8Hz), 7.82 (1 H; t, J=8Hz), 7 82 (1 H, t, J=1 Hz), 8.20-8.28 (1 H, m)

### Preparation 35

The following compounds were obtained according to a similar manner to that of Preparation 2.
(1) Ethyl 3-[3-(3-nitrobenzoyl)indol-1-yl]propionate
   mp: 102-103°C
   NMR (CDCI₃, δ) : 1.20 (3H, t, J=7.5Hz), 2.88 (2H, t, J=6Hz), 4.13 (2H, q, J=7.5Hz), 4.53 (2H, t, J=6Hz), 7.3-7.5 (3H, m), 7.68 (1 H, s), 7.70 (1 H, t, J=₇.₅Hz), 8.4-8.5 (2H, m), 8.62 (1 H; t, J=2Hz)
(2) Ethyl 5-[3-(3-nitrobenzoyl)indol-1-yl]valerate
   mp: 77-78°C
   NMR (CDCl₃, δ) : 1.23 (3H, t, J=7.5Hz), 1.6-1.8 (2H, m), 1.8-2.1 (2H, m), 2.33 (2H, t, J=7Hz), 4.09 (2H; q, J-7.5Hz), 4.22 (2H, t, J=7Hz), 7.3-7.5 (3H, m), 7.58 (1 H, s), 7.71 (1 H, t, J-7.5Hz), 8.17 (1 H, dt, J=2, 7.5Hz), 8.4-8.5 (2H, m), 8.65 (1 H, t, J=2Hz)
(3) Ethyl 4-[5-chloro-3-(3-nitrobenzoyl)indol-1-yl]butyrate
   mp : 111-113°C
   NMR (CDCI₃, δ) : 1.22 (3H, t, J=7.5Hz), 2.10-2.28 (2H, m), 2.32 (2H, t, J=7.5Hz), 4.10 (2H, q, J=7.5Hz), 4.25 (2H, t, J=₇.₅Hz), 7.33 (1 H, dd, J=2.₅, 8Hz), 7.40 (1 H, d, J=8Hz), 7.56 (1 H, s), 7.72 (1 H, t, J=8Hz), 8.15 (1 H, dif-dd, J=8HZ), 3.36-3.46 (2H, m), 8.63 (1 H, dif-d)
(4) Ethyl 4-[2-methyl-3-(3-nitrobenzoyl)indol-1-yl]butyrate
   mp : 92-93°C
   NMR (CDCl₃, δ) : 0.76 (3H, t, J=7.5Hz), 2.05-2.25 (2H, m), 2.44 (2H, t, J=7.5Hz), 2.69 (3H, s), 4.15 (2H, q, J-7.5Hz), 4.25 (2H, t, J=7.5Hz); 7.00-7.28 (3H, m); 7.41 (1 H, d, J=8Hz), 7.65 (1 H, t, J=8Hz), 8.10 (1H, dif-dd, J=8Hz), 8.40 (1H, dif-dd, J=8Hz), 8.58 (1H, dif-d)
(5) Ethyl 4-[3-(3-methoxybenzoyl)indol-1-yl]butyrate
   NMR (CDCI₃, δ) : 1.22 (3H, t, J=7.5Hz), 2.15-2.40 (4H, m), 3.87 (3H, s), 4.12 (2H, q, J=7.5Hz), 4.26 (2H, t, J=7.5Hz), 7.06-7.15 (1H, m), 7 30-7.48 (6H, m), 7.59 (1 H, s), 8.39-8.49 (1 H, m)
(6) Ethyl 4-3-[4-methoxybenzoyl)indol-1-yl]butyrate
   NMR (CDCI₃, δ) : 1.20 (3H, t, J=7.5Hz), 2.08-2.38 (4H, m), 3.38 (3H, s), 4.10 (2H, q, J=7.5Hz), 4.23 (2H, t, J-7.5Hz), 6.99 (2H, d, J=8Hz), 7.28-7.48 (3H, m), 7.58 (1H, s), 7.85 (2H, d, J=8Hz), 8.32-8.45 (1H, m) m), 7.48 (1H, t, J=7.5Hz); 7.61 (1H, d, J=7.5Hz), 8.11 (1H, d, J=8Hz), 8.16 (1 H, broad s), 8.44 (1 H; d, J=8Hz)

### Preparation 36

Aluminum chloride (3.3 g) was added to a solution of ethyl 4-[3-(3-methoxybenzoyl)indolyl-1-yl]butyrate (3.0 g) in a mixture of ethanethiol (10 ml) and dichloromethane (10 ml) at 0°C, and the mixture was stirred at 25°C for 1 hour. After evaporation of the solvent, 1 N hydrochloric acid and ethyl acetate were added to the residue. The mixture was stirred at 25°C for 30 minutes. The organic layer was separated, washed with water and brine, and dried over magnesium sulfate. After evaporation of the solvent, the residue was chromatographed on silica gel (100 g) eluting with chloroform to give ethyl 4-[3-(3-hydroxybenzoyl)indol-1-yl]butyrate (2.65 g) as an oil.
NMR (CDCl₃, 8): 1.22 (3H, t, J=7.5Hz), 2.22-2.38 (4H, m), 4.12 (2H, q, J=7.5Hz), 4.23 (2H, t, J=7.5Hz), 7.00-7.12 (1 H, m), 7.28-7.48 (6H, m), 7.62 (1 H, s), 8.35-8.46 (1 H, m)

### Preparation 37

The following compound was obtained according to a similar manner to that of Preparation 36.

Ethyl 4-[3-(4-hydroxybenzoyl)indol-1-yl]butyrate
mp: 129-131 °C
NMR (CDCl₃, 8): 1.20 (3H, t, J-7.5Hz), 2.08-2.40 (4H, m), 4.10 (2H, q, J-7.5Hz), 6.91 (2H, d, J=8.0Hz), 7.25-7.50 (3H, m), 7.60 (1 H, s), 7.75 (2H, d, J=8.0Hz), 8.30-8.42 (1 H, m)

### Preparation 38

Zinc iodide (0.21 g) was added to a mixture of 4-isobutylbenzaldehyde (6.5 g) and trimethylsilylcyanide (5.0 g). The mixture was stirred at room temperature for 30 minutes and partitioned between ethyl acetate and 7% hydrochloric acid. The organic layer was washed with 7% hydrochloric acid and water and dried over magnesium sulfate. The solvent was removed in vacuo to give 2-hydroxy-2-(4-isobutylphenyl)acetonitrile as a yellow oil (8.15 g).
NMR (CDC|₃, δ) : 0.92 (6H, d, J=7Hz), 1.87 (1 H, m), 2.50 (2H, d, J=7Hz), 5.50 (1 H, s), 7.23 (2H, d, J=9Hz), 7.42 (2H, d, J=9Hz)

### Preparation 39

2-Hydroxy-2-(4-isobutylphenyl)acetonitrile (7.35 g) was added to conc. hydrochloric acid (30 ml). The mixture was refluxed for 2 hours and poured into ice water (100 ml). The organic layer was extracted with ethyl acetate (20 ml), washed with water, dried over magnesium sulfate and evaporated. The residue was crystallized with n-hexane to give 2-hydroxy-2-(4-isobutylphenyl)acetic acid as a white solid (2.75 g).
NMR (CDC|₃, δ) : 7.34 (2H, d, J=9Hz), 7.15 (2H, d, J=9Hz), 5.22 (1 H, s), 2.48 (2H, d, J=7Hz), 1.85 (1 H, m), 0.90 (6H, d, J=₇Hz)

### Preparation 40

To a solution of 2-hydroxy-2-(4-isobutylphenyl)acetic acid (3.8 g) in N,N-dimethylformamide (30 ml) was added potassium carbonate (7.6 g) and benzylbromide (2.2 ml). The mixture was stirred at room temperature for 4.5 hours and poured into ice water and 7% hydrochloric acid. The organic layer was extracted with ethyl acetate, washed with aqueous sodium bircarbonate solution and water, dried over magnesium sulfate and evaporated. The residual white powder was collected with n-hexane by filtration to give benzyl 2-hydroxy-2-(4-isobutylphenyl)acetate as a white powder (4.42 g).
NMR (CDCl₃, δ) : 0.90 (6H, d, J=7Hz), 1.85 (1 H, m), 2,47 (2H, d, J=7Hz), 3.40 (1 H, d, J=6Hz), 5.10 (2H, dd, J=11Hz), 7.1-7.4 (9H, m)

### Preparation 41

A mixture of ethyl 4-[5-chloro-3-(3-nitrobenzoyl)indol-1-yl]butyrate (2.00 g), 1N aqueous sodium hydroxide (7.5 ml), methanol (35 ml) and 1,4-dioxane (35 ml) was stirred at 25°C for 6 hours. After evaporation of the organic solvent, 1 N hydrochloric acid (15 ml) was added to the aqueous solution and the mixture was extracted with ethyl acetate The extract was washed with water, dried over magnesium sulfate, and evaporated in vacuo. The crystalline residue was washed with hot ethanol to give 4-[5-chloro-3-(3-nitrobenzoyl)indol-1-yl]butyric acid (1.77 g) as colorless crystals.
mp : 198-200°C
NMR (DMSO-d₆, δ) : 2.06 (2H, m), 2.30 (2H, t; J=7.5Hz); 4.36 (2H, t, J-7.5Hz), 7.45 (1H, dd, J-2.5, 8Hz), 7.80 (1 H, d, J=8Hz)_{;} 7.91 (1 H, t, J=8Hz), 8.25-8.35 (3H, m), 8.45-8.55 (2H, m)

### Preparation 42

The following compounds were obtained according to a similar manner to that of Preparation 41.
(1) 4-[2-Methyl-3-(3-nitrobenzoyl)indol-1-yl]butyric acid
   mp: 135-138°C
   NMR (CDCl₃, δ) : 2.08-2.25 (2H, m), 3.50 (2H, t, J=7.5Hz)_{;} 2.66 (3H, s), 4.26 (2H, t, J=7.5Hz), 7.00-7.30 (3H, m), 7.49 (1 H, d, J=8.OHz), 7.65 (1 H, t, J=8.OHz), 8.10 (1 H, dif-dd, J=8.OHz), 8.40 (1 H, dif-dd, J=8.OHz), 8.60 (1 H, dif-d)
(2) 4-[3-(3-Hydroxybenzoyl)indol-1-yl]butyric acid
   NMR (DMSO-d₆, δ) : 1.75-1.95 (2H, m), 2.00 (2H, t, J=7.5Hz), 3.97 (2H, t, J=7.5Hz), 6.65-6.77 (1 H, m), 6.88-708 (5H, m), 7.13-7.23 (1 H, m), 7.51 (1 H, s), 800-8.08 (1 H, m)
(3) 4-[3-(4-Hydroxybenzoyl)indolyl-1-yl]butyric acid
   mp: 180-182°C
   NMR (CDCl₃-CD₃0D, δ) : 2.10-2.40 (4H, m), 4.25 (2H, t, J=7.5Hz), 6.90 (2H, d, J=8.OHz), 7.25-7.51 (1 H, m), 7.65 (1 H, s), 7 75 (2H, d, J=8.OHz), 8.25-8.40 (1 H, m)

### Preparation 43

The following compounds were obtained according to a similar manner to that of Preparation 4.
(1) 3-(3-Aminobenzoyl)indole
   NMR (DMSO-d₆, δ) : 6.77 (1 H, dt, J=8, 1 Hz), 6.90 (1 H, dt, J=8, 1 Hz), 6.98 (1 H, t, J=1 Hz), 7.16 (1 H, t, J=8Hz), 7.20-7.35 (2H, m), 7.6-7.5 (1 H, m), 7.90 (1 H, s), 8.2-8.3 (1 H, m)
(2) Ethyl 3-[3-(3-aminobenzoyl)indol-1-yl]propionate
   NMR (CDCI₃, 8): 1.18 (3H, t, J=7.5Hz), 2.86 (2H, t, J=6Hz), 4.10 (2H, q, J=7.5Hz), 4.49 (2H, t, J=6Hz), 6.90 (1 H, dt, J=7.5, 2.5Hz), 7.1-7.4 (6H, m), 7.70 (1 H, s), 8.4-8.5 (1 H, m)
(3) Ethyl 5-[3-(3-aminobenzoyl)indol-1-yl]valerate
   NMR (CDCI₃, δ) : 1.22 (3H, t, J=7.5Hz), 1.6-1.8 (2H, m), 1.8-2.0 (2H, m), 2.32 (2H, t; J=7Hz), 4.11 (2H, q, J=7.5Hz), 4.18 (2H, t, J=7Hz), 6.90 (1 H, d, J=7.5Hz), 7.1-7.5 (6H, m); 7.62 (1 H, s), 8.4-8.5 (1H, m)
(4) 4-[3-(3-Aminobenzoyl)-5-chloroindol-1-yl]butyric acid
   mp: 178-187°C
   NMR (CDCl₃-CD₃OD, δ) : 2.10-2.25 (2H, m), 2 32 (2H, t, J=7.5Hz), 4.26 (2H, t, J=7.5Hz), 6.98 (1H, dif-dd, J=8Hz), 7.15-7.45 (5H, m), 7.70 (1 H, s), 8.40 (1 H, d, J=2.₅Hz)
(5) 4-[3-(3-Aminobenzoyl)-2-methylindol-1-yl]butyric acid
   mp: 138-154°C
   NMR (CDCl₃-CD₃OD, δ) : 2.00-2.20 (2H, m), 2.40 (2H; t, J=7.5Hz), 2.55 (3H, s), 4.22 (2H, t, J=7.5Hz), 6.70-7.00 (1 H, m), 7.00-7.32 (5H, m), 7.32-7.45 (2H, m)
(6) Ethyl 4-[3-(3-aminobenzoyl)indol-1-yl]butyrate
   NMR (CDCI₃, 8): 1.23 (3H, t; J=7Hz), 2.1-2.4 (4H, m), 4.10 (2H, q, J=7Hz), 4.24 (2H, t, J=7Hz), 6.90 (1 H, dt, J=7.5, 2Hz), 7.1-7.5 (6H, m), 7.62 (1 H, s), 8.4-8.5 (1 H, m)

### Example 44

To a solution of 3-(3-aminobenzoyl)indole (1.2 g) in N;N-dimethylformamide (12 ml) were added diisopropylethylamine (1 77 ml) and a solution of bis(4-isobutylphenyl)chloromethane (1.92 g) in N;N-dimethylformamide (5 ml) at 25°C, and the mixture was allowed to stand at the same temperature for 14 hours. The reaction mixture was partitioned between ethyl acetate and water, and the organic layer was washed with water and brine, dried over magnesium sulfate, and evaporated. The residue was chromatographed on silica gel (50 g) eluting with a mixture of ethyl acetate and chloroform (1:20) to give 3-[3-bis(4-isobutylphenyl)methylamino]benzoyl]indole (1.25 g) as pale yellow amorphous powder.
NMR (CDCl₃, 8): 0.90 (12H, d, J=8Hz), 1.74-1.98 (2H, m), 2.46 (4H, d, J=8Hz), 5.48 (1 H, s), 6.75 (1 H, d, J=8Hz), 6.98 (1 H, s), 7.04-7.42 (14H, m), 8.40-8.48 (1 H, m), 8.57 (1 H, broad s)

### Preparation 45

To a solution of 10,11-dihydro-5H-dibenz[b,f]azepine (2.44 g) in tetrahydrofuran (25 ml) was added potassium tert-butoxide (2.02 g) at 0°C. The mixture was stirred at 0°C for 15 minutes, and then a solution of methyl 3-(chloromethyl) benzoate (3.57 g) in tetrahydrofuran (15 ml) was added. The mixture was stirred at 25°C for 1 hour, and then evaporated. The residue was partitioned between ethyl acetate and 0.1 N hydrochloric acid. The organic layer was washed with water and brine, dried over magnesium sulfate, and evaporated. The residue was chromatographed on silica gel eluting with a mixture of hexane and ethyl acetate (40:1) to give methyl 3-(10;11-dihydro-5H-dibenz[b,f]azepin-5-ylmethyl) benzoate (1.10 g) as an oil.
NMR (CDCl₃, δ) : 3.24 (4H, s), 3.39 (3H, s), 5.00 (2H, s), 6.80-6.96 (2H, m), 7.00-7.15 (6H, m), 7 30 (1 H, t, J=8Hz), 7.66 (1 H, dd, J=₁, 8Hz), 7.83 (1 H, dd, J=₁, 1.8Hz), 8.10 (1 H, t, J=1Hz)

### Preparation 46

1 N aqueous solution of sodium hydroxide (9 ml) was added to a solution of methyl 3-(10,11-dihydro-5H-dibenz[b, f]azepin-5-ylmethyl)benzoate (1.06 g) in a mixture of 1,4-dioxane (25 ml) and methanol (15 ml) at 25°C. The mixture was stirred at 25°C for 3 hours, and then at 50°C for 30 minutes. After evaporation of the solvent, the residue was partitioned between ethyl acetate and 0.1 N hydrochloric acid. The organic layer was separated, washed with water and brine, dried over magnesium sulfate, and evaporated to give 3-(10;11-dihydro-5H-dibenz[b,f]azepin-5-ylmethyl) benzoic acid (1.01 g) as powder.
NMR (CDCl₃, δ) : 3.26 (4H, s), 5.02 (2H, s), 6.85-6.96 (2H, m), 7.00-7.20 (6H, m), 7.32 (1 H, t, J=8Hz), 7.64 (1 H, dd, J=1; 8Hz), 7.90 (1H, dd, J=1, 8Hz), 8.17 (1H, t; J=1 Hz)

### Example 1

A mixture of 4-[3-(3-aminobenzoyl)indol-1-yl]butyric acid (880 mg), bis(4-isobutylphenyl)methyl chloride (1.03 g) and diisopropylethylamine (0.945 g) in dichloromethane (20 ml) was stirred at 25°C overnight, and bis(4-isobutylphenyl) methyl chloride (170 mg) and diisopropylethylamine (86 mg) were added thereto. After stirring at 25°C for 3 hours, the reaction mixture was poured into cold 1 N hydrochloric acid. The organic layer was separated, washed with water, and dried over magnesium slat. After evaporation of the solvent, the residue was chromatographed on silica gel (100 g) with a mixture of chloroform and methanol (50:1) as eluent and freeze-dried from benzene to give 4-[3-[3-[bis(4-isobutylphenyl)methylamino]benzoyl]indol-1-yl]butyric acid (820 mg) as pale yellow powder.

NMR (CDCl₃, δ) : 0.89 (12H, d, J=7.5Hz), 1.85 (2H, m), 2.17 (2H, m), 2.28-2.50 (6H, m), 4.20 (2H, t, J=7.5Hz), 5.51 (1 H, s), 6.78 (1 H, broad d), 7.00-7.48 (15H, m); 8.45 (1 H, m)

### Example 2

The following compound was obtained according to a similar manner to that of Example 1. 4-[3-[4-[Bis(4-isobutylphenyl)methylamino]benzoyl]indol-1-yl]butyric acid
NMR (CDCl₃, δ) : 0.92 (12H, d, J=7.5Hz), 1.2-1.9 (2H, m), 2.18 (2H, quintet, J=7.5Hz), 2.38 (2H, t, J=7.5Hz), 2.45 (4H, d, J=₇.₅Hz), 4.22 (2H, t, J=₇.₅Hz), 5.54 (1 H, s), 6.55 (2H, d, J=8Hz), 7.08 (2H, d, J=8Hz), 7.22 (2H, d, J=8Hz), 7.25-7.40 (2H, m), 7.55 (1 H, s), 7.70 (2H, d, J=8Hz), 8.30 (1 H, m)

### Example 3

The following compounds were obtained according to a similar manner to that of Example 1.
(1) Ethyl 3-[3-[3-[bis(4-isobutylphenyl)methylamino]benzoyl]indol-1-yl]propionate
   NMR (CDCI₃, 8): 0.88 (12H, d, J=7Hz), 1.16 (3H, t, J=7.5Hz), 1.84 (2H, m), 2.43 (4H, d, J-7Hz), 2.80 (2H, t, J=7Hz), 4.08 (2H, q, J=7Hz); 4.41 (2H, t, J=7Hz), 5.53 (1 H, s), 6.71 (1 H, d, J=7.5Hz), 7.0-7.4 (6H, m), 7.10 (4H, d, J=8Hz), 7.25 (4H, d, J=8Hz), 7.54 (1 H, s), 8.4-8.5 (1 H, m)
(2) Ethyl 5-[3-[3-[bis(4-isobutylphenyl)methylamino]benzoyl]indol-1-yl]valerate
   NMR (CDCI₃, δ) : 0.89 (12H, d, J=7Hz), 1.19 (3H, t, J=7.5Hz), 1.5-1.7 (2H, m), 1.7-2.0 (4H, m), 2.30 (2H, t, J=₇.₅Hz), 2.45 (4H, d, J=₇Hz), 4.0-4.2 (4H, m), 5.53 (1 H, s), 6.71 (1 H, d, J=₇.₅Hz), 7.09 (4H, d, J=8Hz), 7.25 (4H, d, J=8Hz), 7.0-7.4 (6H, m), 7.46 (1 H, s), 8.4-8.5 (1 H, m)
(3) 4-[3-[3-[Bis(4-isobutylphenyl)methylamino]benzoyl]-5-chloroindol-1-yl]butyric acid
   mp: 150-152°C
   NMR (CDCl₃, δ) : 0.85 (12H, d, J=7.5Hz), 1.70-1.95 (2H, m), 2.05-2.20 (2H, m); 2.31 (2H, m), 2.43 (4H, d, J-7.5Hz), 4.14 (2H, t, J-7.5Hz), 5.50 (1 H, s), 6.70 (1 H, d, J=8Hz), 6.95-7.40 (13H, m), 7.45 (1 H, s), 8.41 (1 H, s)
(4) 4-[3-[3-[Bis(4-isobutylphenyl)methylamlno]benzoyl]-2-methylindol-1 -yl]butyhc acid
   NMR (CDCI₃, δ): 0.85 (12H, d, J=7.5Hz), 1.70-1.93 (2H, m), 2.00-2.20 (2H, m); 2.44-2.52 (9H, m), 4.19 (2H, t, J=7.5Hz),5.49 (1 H, s), 6.63-6.75 (1 H, m), 6.92-7.55 (15H, m)
(5) 4-[3-[3-Bis(3-isobutylphenyl)methylamino]benzoyl]indol-1-yl]butyric acid
   NMR (CDCl₃, δ): 0.78 (12H, d, J=7.5Hz), 1.50-1.82 (2H, m), 1.95-2.18 (2H, m), 2.18-2.40 (6H, m), 4.08 (2H, t, J=7.5Hz), 5.42 (1H, s), 6.61-6.78 (1H, m), 6.90-7.38 (15H, m), 8.28-8.40 (1H, m)
(6) Ethyl 4-[3-[3-[bis(4-isobutylphenyl)methylamino]benzoyl]indol-1-yl]butyrate
   NMR (CDCl₃, δ): 0.88 (12H;d_{;} J=7.5Hz), 1.20 (3H, t, J=8Hz), 1.85 (2H, m), 2.2-2.4 (4H, m), 2.45 (4H, d, J=8Hz), 4.08 (2H, q, J=8Hz); 4.18 (2H, t; J=₇.₅Hz), 5.52 (1 H, s), 6.72 (1 H, d, J=8Hz), 7.0-7.5 (15H, m),8.45 (1H, m)
(7) 4-[3-[3-[1-(4-Isobutylphenyl)ethylamino]benzoyl]indol-1-yl]butyric acid
   NMR (CDCl₃, δ): 8.43 (1H, m), 7.45 (1H, s), 7.0-7.4 (10H, m), 6.67 (1H, d; J=7.5Hz)_{;} 4.52 (1H, q, J=₇Hz), 4.20 (2H, m), 2.43 (2H, d, J=7.5Hz), 2.35 (2H, m), 2.1-2.3 (2H, m), 1.7-2.0 (1H, m), 1.51 (3H, d, J=7Hz), 0.90 (6H, d, J=7.5Hz)
(8) 4-[3-[4-[1-(4-Isobutylphenyl)ethylamino]benzoyl]indol-1-yl]butyric acid
   NMR (CDCI₃, δ): 8.30 (1 H, m), 7.70 (2H, d, J=9Hz), 7.51 (1 H, s), 7.2-7.5 (5H, m); 7.10 (2H; ξd, J=9Hz), 6.5-6.7 (2H, m), 4.56 (1H, q, J=7Hz), 4.23 (2H, m), 2.45 (2H, d, J=7.5Hz), 2.35 (2H, m), 1.7-2.0 (1 H, m), 1.60 (3H, d, J=₇Hz), 0.87 (6H, d, J=₇.₅Hz)

### Example 4

A mixture of ethyl 4-[3-(3-aminobenzoyl)indol-1-yl]butyrate (1.0 g) and p-isobutylbenzyl chloride (1.56 g) and potassium carbonate (1.18 g) in N,N-dimethylformamide (20 ml) was heated at 100°C for 23 hours. More p-isobutylbenzyl chloride (0.52 g) and potassium carbonate (0.394 g) were added, and the mixture was heated at 100°C for 30 minutes. The reaction mixture was poured into ice water and was extracted with ethyl acetate. The extract was washed with water, and dried over magnesium sulfate. After evaporation of the solvent, the residue was chromatographed on silica gel (100 g) eluting with a mixture of ethyl acetate and hexane (1:4 → 1:3) to give ethyl 4-[3-[3-[bis(4-isobutylbenzyl) amino]benzoyl]indol-1-yl]butyrate (1.06 g, Compound I) and ethyl 4-[3-[3-(4-isobutylbenzyl)aminobenzoyl]indol-1-yl] butyrate (184 mg, Compound II).

### Compound I

NMR (CDCl₃, δ): 0.90 (12H, d, J=7Hz), 1.20 (3H, t, J-7Hz), 1.84 (2H, m), 2.10 (2H, quin, J=7Hz), 2.23 (2H, t, J=7Hz), 2.45 (4H, d, J=7Hz), 4.0-4.2 (4H, m), 4.67 (4H, s), 6.92 (1 H, broad d, J=7.5Hz), 7.0-7.5 (15H, m), 8.4-8.5 (1 H, m)

### Compound II

NMR (CDCl₃, δ): 0.90 (6H, d, J=7Hz), 1.21 (3H, t, J=7Hz), 1.85 (1 H, m), 2.1-2.4 (4H, m), 2.46 (2H, d, J=7Hz), 4.10 (2H, q, J=₇Hz), 4.21 (2H, t, J=₇Hz), 4.35 (2H, s), 6.82 (1H, d, J=₇.₅Hz), 7.1-7.5 (10H, m), 7.57 (1 H, s), 8.4-8.5 (1 H, m)

### Example 5

A solution of 4-[3-(3-hydroxybenzoyl)indol-1-yl]butyric acid (500 mg) in N,N-dimethylformamide (5 ml) was added to a suspension of sodium hydride (60% dispersion in mineral oil, 136 mg) in N,N-dimethylformamide (10 ml) at 25°. The mixture was stirred at 25°C for 1 hour, and a solution of bis (4-isobutylphenyl)bromomethane (1.11 g) in tetrahydrofuran (10 ml) was added at 0°C. After stirred at 25°C overnight, the mixture was poured into a mixture of ethyl acetate and 1 N hydrochloric acid. The organic layer was separated, washed with water and brine, and dried over magnesium sulfate. After evaporation of the solvent, the residue was chromatographed on silica gel (20 g) eluting with 2% methanol in chloroform to give 4-[3-[3-[bis(4-isobutylphenyl)methoxy]benzoyl]indol-1-yl]butyric acid (120 mg) as colorless crystals.
mp: 154-156°C
NMR (CDCl₃, δ): 0.88 (12H, d, J=7.5Hz), 1.70-1.95 (2H, m), 2.08-2.26 (2H, m), 2.34 (t, 2H, J=7.5Hz), 2.44 (4H, d, J=8Hz), 4.20 (2H, t, J=7.5Hz), 6 26 (1 H, s), 6.90-7.40 (15H, m), 7.44 (1 H, s), 8.37-8.45 (1 H, m)

### Example 6

The following compounds were obtained according to a similar manner to that of Example 5.
(1) 4-[3-[4-[Bis(4-isobutylphenyl)methoxy]benzoyl]indol-1-yl]butyric acid
   NMR (CDCI₃, δ): 0.88 (12H, d, J-7.5Hz), 1.79-1.98 (2H, m), 2.10-2.30 (2H, m); 2.30-2.50 (6H, m), 4.25 (2H, t, J=7.5Hz), 6.28 (1H, s), 7.02 (2H, d, J=8.0Hz), 7.12 (4H, d, J=8Hz), 7.20-7.45 (7H, m), 7.55 (1 H, s), 7.75 (2H; d, J=8Hz), 8.28-8.40 (1 H, m)
(2) 4-[3-[4-[1-(4-Isobutylphenyl)ethoxy]benzoyl]indol-1-yl]butyric acid
   NMR (CDCI₃, δ): 0.90 (6H, d, J=7.5Hz), 1.69 (3H, d, J=7.5Hz), 1.75-1 98 (1 H, m), 2.12-2.32 (2H, m), 2 32-2.52 (4H, m), 4.29 (2H, t, J=₇.₅Hz), 6.98 (2H, d, J=8Hz), 7.15 (2H, d, J=8Hz), 7.25-7.50 (5H, m), 7.89 (2H, d, J=8Hz), 8.30-8.42 (1 H, m)

### Example 7

A mixture of 3-[3-[2,2-bis(4-isobutylphenyl)ethyl]benzoyl]indole (442 mg), ethyl 4-bromobutyrate (185 mg) and potassium carbonate (360 mg) in N,N-dimethylformamide (10 ml) was stirred at 20°C for 4 hours. Ethyl 4-bromobutyrate (185 mg) and potassium carbonate (120 mg) were added, and resulting mixture was stirred at 20°C for 16 hours. The reaction mixture was poured into a mixture of ethyl acetate and 1 N-hydrochloric acid. The organic layer was separated, washed with water and brine, and dried over magnesium sulfate. After evaporation of the solvent, the residue was purified by column chromatography on silica gel (20 g) eluting with a mixture of hexane and ethyl acetate (10:1) to give ethyl 4-[3-[3-[2,2-bis(4-isobutylphenyl)ethyl]benzoyl]indol-1-yl]butyrate (480 mg) as an oil.
NMR (CDCl₃, δ): 0.82 (12H, d, J=7.5Hz), 1.18-1.48 (3H, m), 1.70-1.94 (2H, m), 2.15-2.50 (8H, m), 3.45 (2H, d, J=7.5Hz); 4.00-4.35 (5H, m), 6.95-7.70 (16H, m), 8.35-8.49 (1 H, m)

### Example 8

The following compounds were obtained according to a similar manner to that of Example 9.
(1) Ethyl 4-[3-[4-[2,2-bis(4-isobutylphenyl)ethyl]benzoyl]indol-1 -yl]butyrate
   NMR (CDCI₃, σ): 0.88 (12H, d, J=7.5Hz), 1.16-1.32 (3H, m), 1.72-1.92 (2H, m), 2.08-2.58 (8H, m), 3.36-3.54 (4H, m),, 4.03-4.32 (3H, m), 6.98-7.18 (10H, m), 7.29-7.48 (3H, m), 7.51 (1 H, s), 7.63 (2H, d, J=B.OHz), 8.30-8.41 (1 H, m)
(2) Ethyl 4-[3-[3-[1-(3-isobutylphenyl)ethoxy]benzoyl]indol-1-yl]butyrate
   NMR (CDCI₃, σ): 0.83 (3H, d, J=8Hz), 0.85 (3H, d, J=8Hz), 1.20 (3H, t, J=8Hz), 1.65 (3H; d, J=8Hz), 1.70-1.92 (1 H, m), 2.08-2.23 (2H, m), 2.28 (2H, t, J=8Hz), 244 (2H, d, J=8Hz), 4.06-4.32 (2H, m), 4.10 (2H, q, J=8Hz), 5.36 (1 H, q, J=8Hz), 7.00-7.42 (12H, m), 8.36-8.47 (1 H, m)
(3) Ethyl 4-[3-[3-[bis(4-isobutylphenyl)methylamino]-benzoyl]indol-1-yl]-2-butenoate
   NMR (CDCI₃, δ): 0.88 (12H, d, J=8Hz), 1.30 (3H, t, J=7Hz), 1.73-1.96 (2H, m), 2.44 (4H, d, J=8Hz), 3.28 (2H, dd, J=1, 7Hz), 4.20 (2H, q, J=7Hz), 5.54 (1 H; s), 6 00 (1 H, dt, J=14, 7Hz), 6.73 (1 H, broad d, J=8Hz), 7.0-7.4 (14H, m), 7.45-7.52 (1 H, m), 7.76 (1 H, s), 8.36-8.44 (1 H, m)
(4) Ethyl 4-[3-[3-(10,11 -dihydro-5H-dibenz[b,f]azepin-5-ylmethyl)benzoyl]indol-1 -yl]butyrate
   NMR (CDCI₃, δ): 1.20 (3H, t, J=8Hz), 2.08-2.22 (2H, m), 2.30 (2H, t, J=8Hz), 3.22 (4H, s), 4.08 (2H, q, J=8Hz), 4.20 (2H, t, J=8Hz), 5.05 (2H, s), 6.85-6.96 (2H, m), 7.04-7.20 (5H, m), 7.25-7.50 (6H, m), 7.62 (2H, t, J=8Hz), 7.86 (1 H; t, J=₁ Hz), 8.35-8.47 (1 H, m)

### Example 9

A mixture of ethyl 4-[3-(4-hydroxybenzoyl)indol-1-yl]butyrate (0.50 g), 1-bromo-2,2-dimethyl-1-(4-isobutylphenyl) propane (0.74 g) and potassium carbonate (0.59 g) in N,N-dimethylformamide (10 ml) was stirred at 60°C for 20 hours. The reaction mixture was filtered, and the filtrate was poured into a mixture of ethyl acetate and 0.5N hydrochloric acid. The organic layer was separated, washed with water, and dried over magnesium sulfate. After evaporation of the solvent, the residue was purified by column chromatography on silica gel eluting a mixture of n-hexane and ethyl acetate (5:1) to give ethyl 4-[3-[4-[2,2-dimethyl-1-(4-isobutylphenyl)propyloxy]benzoyl]indol-1-yl]butyrate (376 mg) as an oil.
NMR (CDCl₃, δ): 0.88 (6H, d, J=7Hz), 1.03 (9H, s), 1.20 (3H; t, J=7.5Hz), 1.74-1.96 (1 H, m); 2.10-2.35 (4H, m), 2.45 (2H, d, J=₇Hz), 4.10 (2H, q, J=₇.₅Hz), 4.22 (2H, t, J=₇Hz), 4.81 (1 H, s), 6.88 (2H, d, J=9Hz), 7.08 (2H, d, J=8.5Hz), 7.20-7.45 (5H, m), 7.51 (1 H, s), 7.70 (2H, d, J=9Hz), 8.31-8.41 (1 H, m)

### Example 10

The following compound was obtained according to a similar manner to that of Example 9. Ethyl 4-[3-[4-[1-(4-isobutylphenyl)propoxy]benzoyl] indol-1-yl]butyrate
NMR (CDCl₃, δ): 0.89 (6H, d, J=7Hz), 1.02 (3H, t, J=7.5Hz), 1.20 (3H, t, J=7.5Hz), 1.75-2.10 (3H, m), 2.15-2.35 (4H, m), 2.46 (2H, d, J=7Hz), 4.10 (2H, q, J-7.5Hz), 4.23 (2H, t, J-7Hz), 5.10 (1H, t; J=7Hz), 6.93 (2H, d, J=9Hz), 7.12 (2H, d, J=8.5Hz), 7.22-7.45 (5H, m), 7.53 (1 H, s), 7.75 (2H, d, J=9Hz), 8.32-8.40 (1 H, m)

### Example 11

To a solution of ethyl 3-[3-[3-[bis(4-isobutylphenyl)methylamino]benzoyl]indol-1-yl]propionate (507 mg) in a mixture of 1,4-dioxane (8 ml) and methanol (2 ml) was added 1 N aqueous sodium hydroxide (2 ml), and the mixture was stirred at 25°C for 5 hours. After evaporation of the organic solvent, the residue was acidified (pH 2) with 1N hydrochloric acid, and extracted with ethyl acetate. The extract was washed with water and dried over magnesium sulfate. After evaporation of the solvent, the residue was chromatographed on silica gel (20 g) eluting with chloroform to give 3-[3-[3-[bis(4-isobutylphenyl)methylamino]benzoyl]indol-1-yl]propionic acid (375 mg) as an oil.
NMR (CDCl₃, δ): 0.88 (12H, d, J=7Hz), 1.82 (2H, m), 2.42 (4H, d, J=7Hz), 2.85 (2H, t, J=6Hz), 4.38 (2H, t, J=6Hz), 5.52 (1 H, s), 6.71 (1 H, d; J=7.5Hz), 7.0-7.4 (6H, m), 7.08 (4H, d, J=8Hz), 7.25 (4H, d, J=8Hz), 7.52 (1 H, s), 8.4-8.5 (1 H, m)

### Example 12

The following compounds were obtained according to a similar manner to that of Example 11.
(1) 5-[3-[3-[Bis(4-isobutylphenyl)methylamino]benzoyl]indol-1-yl]valeric acid
   NMR (CDCI₃, δ): 0.87 (12H, d, J=7Hz), 1.5-1.7 (2H, m), 1.7-2.0 (4H, m), 2.34 (2H, t, J=6Hz), 2.44 (4H, d, J=₇Hz), 4.10 (2H, t, J=6Hz), 5.5 (1 H, s), 6.71 (1 H, broad d, J=₇.₅Hz), 7.0-7.4 (6H, m), 7.10 (4H, d, J=8Hz), 7.25 (4H, d, J=8Hz), 7.48 (1 H, s), 8.4-8.5 (1 H, m)
(2) 4-[3-[3-[(4-isobutylbenzyl)amino]benzoyl]indol-1 -yl]butyric acid
   NMR (CDCI₃, δ): 0.88 (6H, d, J=7Hz), 1.84 (1 H, m), 2.17 (2H, quin, J=7Hz), 2.35 (2H, t, J=7Hz), 2.45 (2H, d, J=₇Hz), 4.21 (2H; t, J=₇Hz), 4.33 (2H, s), 6.82 (1 H, d, J=₇.₅Hz), 7.0-7.4 (10H, m), 7.55 (1 H, s), 8.4-8.5 (1 H, m)
(3) 4-[3-[3-[Bis(4-isobutylphenyl)methylamino]benzoyl]indol-1-yl]butyric acid
   NMR (CDCI₃, δ): 0.89 (12H, d, J=7.5Hz), 1.85 (2H, m), 2.17 (2H, m), 2.28-2.50 (6H, m), 4.20 (2H, t, J=7.5Hz), 5.51 (1H, s), 6.78 (1H; broad d), 7.00-7.48 (15H, m), 8.45 (1H, m)
(4) 4-[3-[3-[2,2-Bis(4-isobutylphenyl)ethyl]benzoyl]indol-1 -yl]butyric acid
   NMR (CDCI₃, δ): 0.82 (12H, d, J=7.5Hz), 1.65-1.96 (2H, m), 2.10-2 30 (2H, m), 2 30-2.45 (6H, m), 3.39 (2H, d, J=₇.₅Hz), 4.15-4.32 (3H, m), 7.00 (4H, d, J=8.0Hz), 7.10 (4H, d, J=8.0Hz), 7.18-7.48 (6H, m), 7.48-7.62 (2H, m), 8.30-8.42 (1 H, m)
(5) 4-[3-[4-[2,2-Bis(4-isobutylphenyl)ethyl]benzoyl]indol-1 -yl]butyric acid
   NMR (CDCI₃, δ): 0.88 (12H, d, J=7.5Hz), 1.70-1.92 (2H, m), 2.10-2.30 (2H, m), 2.30-2.50 (6H, m), 3.38 (2H, d, J=₇.₅Hz), 4.10-4.34 (3H, m), 6.93-7.18 (10H, m), 7.28-7.45 (3H, m), 7.50 (1H, s), 7.62 (2H, d, J=B.OHz), 8.30-840 (1 H, m)
(6) 4-[3-[4-[2,2-Dimethyl-1-(4-isobutylphenyl)propyloxy]benzoyl]indol-1-yl]butyric acid
   NMR (CDCI₃, δ): 0.87 (6H, d, J=7Hz), 1.02 (9H, s), 1.83 (1H, m), 2.18 (2H, m), 2.38 (2H, m), 2.43 (2H, d, J-7Hz), 4.21 (2H, t; J-7Hz), 4.79 (1 H, s), 6.88 (2H, d, J=8Hz), 7.07 (2H, d, J=8Hz), 7.21 (2H, d, J=8Hz), 7.2-7.5 (3H, m), 7.52 (1 H, s), 7 70 (2H, d, J=8Hz), 8 32 (1 H, m)
(7) 4-[3-[4-[1-(4-Isobutylphenyl)propoxy]benzoyl]indol-1-yl]butyric acid
   NMR (CDCl₃, δ): 0.88 (6H, d, J=7Hz), 1.00 (3H, t, J=7.5Hz), 1.75-2.10 (3H, m), 2.12-2.28 (2H, m), 2.32-2.50 (4H, m), 4.23 (2H, t, J=7Hz), 5.08 (1 H, t, J-7Hz), 6.92 (2H; d, J-9Hz), 7.11 (2H, d, J=8.5Hz), 7.13-7.45 (5H, m);7.54 (1H, s), 7.73 (2H, d, J=9Hz), 8.38-8.48 (1 H, m)
(8) 4-[3-[3-[1-(3-Isobutylphenyl)ethoxy]benzoyl]indol-1-yl]butyric acid
   NMR (DMSO-d₆, δ): 0.70 (3H, d; J=8Hz), 0.74 (3H, d, J=8Hz), 1.60 (3H, d, J=8Hz), 1.62-1.80 (1H, m), 1.90-2.10 (2H, m), 2.40 (2H, d, J=8Hz), 4.30 (2H, m), 5.58 (1H, q, J=8Hz), 7.00-7.45 (10H, m), 7.75 (1 H, d, J=8Hz); 7.87 (1 H, s), 8.24 (1 H, dd, J=2, 8Hz)
(9) (E)-4-[3-[3-[Bis(4-isobutylphenyl)methylamino]benzoyl]indol-1 -yl]-2-butenoic acid
   NMR (DMSO-d₆, δ): 0.83 (12H, d, J=8Hz), 1.62-1.90 (21 H, m), 2.38 (4H, d, J=8Hz); 3.27 (2H, d, J=7Hz), 5.65 (1 H, d, J=7Hz); 6.24 (1 H, dt, J=14, 7Hz), 6.87 (1 H, d, J=8Hz), 6.97 (1 H, d, J=8Hz), 7.05-7.50 (13H, m), 7.78 (1 H, d, J=8Hz), 8.24 (1H; d, J=8Hz)
(10) 4-[3-[3-[N-Benzyl-N-[1-(4-isobutylphenyl)propyl]amino]benzoyl]indol-1-yl]butyric acid
   NMR (CDCI₃, δ): 0.87 (6H, d, J=7Hz), 1.05 (3H, t, J=7Hz), 1.7-1.9 (1 H, m), 2.0-2.2 (4H, m), 2.3-2.5 (4H, m), 4.08 (2H, t, J=7Hz), 4.37 (2H, d, J=8Hz), 5.04 (1 H, t, J=7Hz), 7.0-7.4 (17H, m), 8.4-8.5 (1 H, m)
(11) 4-[3-[3-[N-[1-(4-Isobutylphenyl)propyl-N-ethylamino]benzoyl]indol-1-yl]butyric acid
   NMR (CDCl₃-CD₃OD, δ): 0.89 (6H, d, J=7Hz), 0.95-1.10 (6H, m), 1 75-2.41 (2H, m), 2.46 (2H, d, J=7Hz), 3.27 (2H, q, J=₇Hz), 4.50 (2H, t, J=₇Hz), 4.90 (2H, t, J=₇Hz), 7.05-7.60 (10H, m), 7.62 (1 H, s), 7.75 (1 H, s), 8.30-8.40 (1 H, m)
(12) 4-[3-[3-[N-[1-(4-Isobutylphenyl)propyl]-N-methylamino]benzoyl]indol-1-yl]butyric acid
   NMR (CDCI₃, δ): 0.90 (6H, d, J=7Hz), 0.98 (3H, t, J=7Hz), 1.72-2.28 (5H, m), 2.30-2.50 (4H, m), 2.75 (3H, s), 4.23 (2H, t, J=7Hz); 4.89 (1 H, t, J=₇Hz), 6.97-7.48 (11 H, m), 7.62 (1 H, s), 8.40-8.50 (1 H, m)
(13) 4-[3-[3-[N-[2,2-Dimethyl-1-(4-isobutylphenyl)propyl]-N-methylamino]benzoyl]indol-1-yl]butyric acid
   NMR (CDCI₃, δ): 0.87 (6H, d, J=7Hz), 1.17 (9H, s), 1.72-1.94 (1 H, m), 2.10-2.28 (2H, m), 2.32-2.47 (4H, m), 2.90 (3H, s), 4.24 (2H, t, J=₇Hz), 4.79 (1 H, s), 6.98-7.46 (11 H, m), 7.62 (1H, s), 8.40-8.50 (1H,m)
(14) 4-[3-[3-[N-[Bis(4-isobutylphenyl)methyl]-N-methylamino]benzoyl]indol-1-yl]butyric acid
   NMR (CDCl₃, δ): 0.89 (12H, d, J=7Hz), 1.73-1.97 (2H, m), 2.07-2.26 (2H, m), 2.37 (2H, t, J=7Hz), 2.45 (4H, d, J=7Hz), 2.78 (3H; s), 4.20 (7H, t, J-7Hz), 6.19 (1H, s); 6.90-7.20 (10H, m), 7.25-7.42 (5H, m), 7.54 (1 H, s), 8.40-8.50 (1 H, m)
(15) 4-[3-[3-[N-Benzyl-N-[1-(4-isobutylphenyl)ethyl]amino]benzoyl]indol-1-yl]butyric acid
   NMR (CDCI₃, δ): 0.88 (6H, d, J=7Hz), 1.59 (3H; d, J=7Hz), 1.70-1.94 (1H, m), 2.00-2.20 (2H, m), 2.31 (2H, t, J=7Hz), 2.42 (2H, d, J=7Hz), 3.95-4.22 (2H, m), 4.48 (2H, s), 5.34 (1H, q, J=7Hz), 6.90-7.42 (17H, m), 8.38-8.48 (1 H, m)
(16) 4-[3-[3-(10,11 -Dihydro-5H-dibenz[b,f]azepin-5-yl-methyl)benzoyl]indol-1 -yl]butyhc acid
   NMR (DMSO-d₆, δ): 1.9-2.1 (2H, m), 2.20 (2H, t, J=8Hz); 3.60 (4H, s), 4.30 (2H, t, J=8Hz), 5.05 (2H, s), 6.89 (2H, t, J=8Hz), 7.04-7 70 (12H, m), 7.82 (2H, d, J=10Hz), 8.26 (1H, dd, J=1, 10Hz)

### Example 13

To a solution of 4-[3-[3-[bis(4-isobutylphenyl)-methylamino]benzoyl]indol-1-yl]butyric acid (3.60 g) in ethanol (30 ml) was added a aqueous solution of sodium bicarbonate (7 ml). After filtration, the solvent was removed in vacuo to give a yellow solid of sodium 4-[3-[3-[bis (4-isobutylphenyl)methylamino]benzoyl-indol-1-yl]butyrate (3.68 g).
mp: 118-125°C
NMR (DMSO-d₆, δ): 0.83 (12H; d, J=7.5Hz), 1.78 (2H, m), 1.94 (4H, m), 2.38 (4H, d, J=7.5Hz), 4.25 (2H, m), 5.65 (1 H, d, J=₇.₅Hz), 5.75 (1 H, d, J=₇.₅Hz), 6.8-7.0 (2H, m), 7.10 (2H, d, J=8Hz), 7.35 (2H, d, J=8Hz), 7.1-7.3 (3H, m), 7.68 (1 H, m), 7.80 (1 H, s), 8.25 (1 H, m)

### Example 14

To a solution of 4-[3-[3-[bis(4-isobutylphenyl)-methylamino]benzoyl]indol-1-yl]butyric acid (150 mg) and diisopropylethylamine (129 mg) in dichloromethane (3 ml) was added benzylbromide (171 mg). The mixture was stirred at room temperature for 3 days and evaporated The residue was dissolved in ethyl acetate The solution was washed with 0.5N hydrochloric acid and water, dried over magnesium sulfate and evaporated. The residue was chromatographed on silica gel (10 g) eluting with a mixture of n-hexane and ethyl acetate (5:1) to give benzyl 4-[3-[3-[N-benzyl-N-[bis(4-isobutylphenyl)methyl]amino]benzoyl]indol-1-yl]butyrate (30 mg) as powder.
NMR (CDCl₃, δ): 0.83 (12H, d, J-7Hz), 1.6-1.9 (2H, m), 2.0-2.15 (2H, m), 2.28 (2H, t, J-7Hz), 2.39 (4H, d, J=7Hz), 4.00 (2H, t, J=7Hz), 4.62 (2H, s), 5.09 (2H, s), 6.38 (1 H, s), 6.9-7.4 (26H, m), 8.4-8.5 (1 H, m)

### Example 15

4N Hydrochloric acid in 1,4-dioxane (2 ml) was added to a solution of benzyl 4-[3-[3-[N-benzyl-N-(tert-butoxycarbonyl)amino]benzoyl]indol-1-yl]butyrate (0.31 g) in 1,4-dioxane (2 ml). The mixture was stirred at room temperature for 1 hour and evaporated. The residue was dissolved in ethyl acetate. The solution was washed with sodium bicarbonate aqueous solution and brine, dried over magnesium sulfate and evaporated to give benzyl 4-[3-(3-benzylami- nobenzoyl)indol-1-yl]butyrate (258 mg) as an oil.
NMR (CDCl₃, δ): 2.1-2.3 (2H, m), 2.37 (2H, t, J=7Hz), 4.17 (2H, t, J-7Hz), 4.39 (2H, s), 5.08 (2H, s), 6.8-6.9 (1 H, m), 7 1-7.45 (16H, m), 7.49 (1 H, s), 8.4-8.5 (1 H, m)

### Example 16

To a solution of benzyl 4-[3-(3-benzylaminobenzoyl)indol-1-yl]butyrate (88 mg) and diisopropylethylamine (45 mg) in dichloromethane (3 ml) was added a solution of 1-bromo-1-(4-isobutylphenyl)propane (90 mg) in dichloromethane (1 ml). The mixture was stirred at room temperature for 24 hours and evaporated. The residue was dissolved in ethyl acetate. The solution was washed with water, dried over magnesium sulfate and evaporated. The residue was chromatographed on silica gel (5 g) eluting with a mixture of n-hexane and ethyl acetate (4:1) to give benzyl 4-[3-[3-[N-benzyl-N-[1-(4-isobutylphenyl)propyl]amino]benzyl]indol-1-yl]butyrrate (30 mg) as an oil.
NMR (CDCl₃, δ): 0.85 (6H, d, J=7Hz), 1.03 (3H, t, J=7Hz), 1.7-1.9 (1 H, m), 2.0-2.2 (4H, m), 2.31 (2H, t, J=7Hz), 2.41 (2H, d, J=₇Hz), 4.06 (2H, t, J=₇Hz), 4.37 (2H, d, J=8Hz); 5.0-5.1 (3H, m), 7.0-7.4 (22H, m), 8.4-8.5 (1 H, m)

### Example 17

The following compounds were obtained according to a similar manner to that of Example 16.
(1) Ethyl 4-[3-[3-[N-[1 -(4-isobutylphenyl)propyl]-N-ethylamino]benzoyl]indol-1 -yl]butyrate
   NMR (CDCI₃, 8): 0.88 (6H, d, J=7Hz), 0.95-1.08 (6H, m), 1.22 (3H, t, J=7Hz), 1.72-2.47 (7H, m), 2.45 (2H, d, J=7Hz), 3.20 (2H, q, J=7Hz), 4.11 (2H, q, J=7Hz), 4.23 (2H, t, J=7Hz), 4 91 (1 H, t, J=7Hz), 6.97-7.50 (1 H, m), 7.63 (1 H, s), 8.40-8.50 (1 H, m)
(2) Methyl 4-[3-[3-[N-[1 -(4-isobutylphenyl)propyl]-N-methylamino]benzoyl]indol-1 -yl]butyrate
   NMR (CDCI₃, δ): 0.89 (6H, d, J=7Hz), 1.00 (3H, t, J=7Hz, 1.74-2.38 (7H, m), 2.43 (2H, d, J=7Hz), 2.75 (3H, s), 3.65 (3H, s), 4.22 (2H, t, J=7Hz), 4.91 (1 H, t, J=7Hz), 6.98-7.45 (11 H, m), 7.62, (1 H, s), 8.40-8.50 (1 H, m)
(3) Ethyl 4-[3-[3-[N-[2,2-dimethyi-1 -(4-isobutylphenyl)propyl]-N-methylamino]benzoyl]indol-1 -yl]butyrate
   NMR (CDCl₃, σ): 0.88 (6H, d, J=7Hz), 1.14-1.28 (9H, m); 1.72-1.95 (1 H, m), 2.10-2.36 (4H, m), 2.42 (2H, d, J=7Hz), 2.90 (1 H, s), 4.10 (2H, q, J=7Hz), 4.23 (2H, t, J=7Hz); 4.79 (1 H, s), 6.98-7.47 (11 H, m), 7.62 (1 H, s), 8.40-8.50 (1 H, m)
(4) Ethyl 4-[3-[3-[N-[bis(4-isobutylphenyl)methyl]-N-methylamino]benzoyl]indol-1-yl]butyrate
   NMR (CDCI₃, σ): 0.90 (12H, d, J=7Hz), 1 20 (3H, t, J=7Hz), 1.75-1.98 (2H, m), 2.08-2.34 (4H, m), 2.46 (4H, d, J=₇Hz), 2.79 (3H, s), 4.03-4.26 (4H, m), 6.19 (1 H, s), 6.92-7.20 (10H, m), 7.25-7.45 (5H, m), 7.55 (1 H, s), 8.40-8.50 (1 H, m)
(5) Benzyl 4-[3-[3-[N-benzyl-N-[1-(4-isobutylphenyl)ethyl]amino]benzoyl]indol-1-yl]butyrate
   NMR (CDCI₃, σ): 0.88 (6H, d, J=7Hz), 1.61 (3H, d, J=7Hz), 1.72-1.95 (1 H, m), 2.00-2.20 (2H, m), 2.30 (2H, t, J=₇Hz), 2.43 (2H, d, J=₇Hz), 4.04 (2H, m), 4.47 (2H, s), 5.09 (2H, s), 5.35 (1 H, t, J=₇Hz), 6.9-7.4 (22H, m), 8.38-8.48 (1 H, m)

### Example 18

Benzyl 4-[3-[3-[N-benzyl-N-[bis(4-isobutylphenyl)methyl]amino]benzoyl]indol-1-yl]butyrate (24 mg) was dissolved in a mixture of methanol 93 ml) and 1,4-dioxane (3 ml), and 10% palladium on carbon (12 mg) was added. The mixture was stirred under hydrogen atmosphere at room temperature for 5 hours. The catalyst was removed by filtration and the filtrate was evaporated. The residue was chromatographed on silica gel (2 g) eluting with a mixture of chloroform and methanol (50: 1) to give 4-[3-[3-[N-benzyl-N-[bis(4-isobutylphenyl)methyl]amino]benzoyl]indol-1-yl]butyric acid as (14 mg) powder.
NMR (CDCl₃, δ): 0.82 (12H, d, J-7Hz), 1.7-1.9 (2H, m), 2.0-2.2 (2H, m), 2.2-2.45 (6H, m); 4.03 (2H, t; J=7Hz), 4.63 (2H, s), 6.38 (1 H, s), 6.9-7.4 (21 H, m), 8.4-8.5 (1 H, m)

### Example 19

A solution of 4N-hydrogen chloride (1.5 ml) in ethyl acetate was added to a solution of 4-[3-[3-[bis(4-isobutylphenyl) methylamino]benzoyl]indol-1-yl]butyric acid (3.0 g) in ethyl acetate (15 ml). The mixture was refrigerated for 16 hours to give 4-[3-[3-[bis(4-isobutylphenyl)methylamino]benzoyl]indol-1-yl]butyric acid hydrochloride as yellow crystals (3.1 g)^{.}
NMR (DMSO-d₆, δ): 0.80 (12H, d, J=7Hz), 1.7-1.9 (2H, m), 1.9-2.1 (2H, m), 2.1-2.3 (2H, m), 2.40 (4H, d, J=7HZ) 4.2-4.4 (2H, m), 5.67 (1 H, s), 6.9-7.4 (6H, m), 7.12 (4H, d, J=8Hz), 7.33 (4H, d, J=8Hz), 7.62 (1 H, m), 7.88 (1 H, s), 8.25 (1 H, m)

## Claims (Claims for the following Contracting State(s) : AT, BE, CH, DE, DK, FR, GB, IT, LI, LU, NL, SE)

1. Compound of the formula: 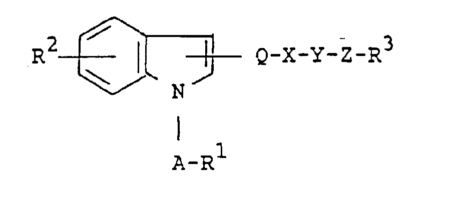 wherein
R¹ is carboxy or protected carboxy,
R² is hydrogen or C₁- C₆ alkyl or halogen,
R³ is mono-or diphenyl(C₁-C₆)-alkyl which may be substituted by C₁-C₆ alkyl, or 10,11-dihydro-5H-dibenzo [b,f]azepinyl,
A is C₁-C₆ alkylene,
Q is carbonyl,
X is 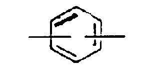
Y is bond,
Z is C₁- C₆ alkylene, -O- or 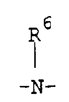
in which R⁶ is hydrogen or C₁- C₆ alkyl or phenyl(C₁- C₆)alkyl,
and pharmaceutically acceptable salts thereof.

2. A compound of claim 1, which is represented by the formula: 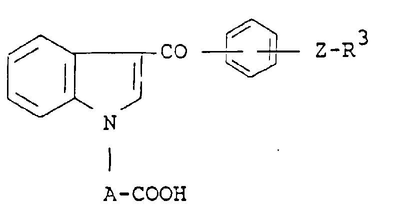 wherein R^{3.} A and Z are each as defined in claim 1.

3. A compound of claim 2, which is selected from the group consisting of:
4-[3-[3-[bis(4-isobutylphenyl)methylamino]benzoyl]indol-1-yl]butyric acid,
4-[3-[4-[bis(4-isobutylphenyl)methoxy]benzoyl]indol-1-yl]butyric acid,
4-[3-[4-[1 -(4-isobutylphenyl)ethoxy]benzoyl]indol-1 -yl]butyric acid and
4-[3-[3-[2,2-bis(4-isobutylphenyl)ethyl]benzoyl]indol-1-yl]butyric acid.

4. A process for preparing a compound of the formula: 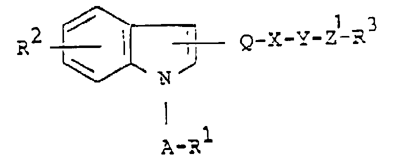 wherein R¹; R², R³, A, Q, X and Y are each as defined in Claim 1, Z¹ is -O- or 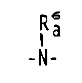
in which R 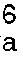 is hydrogen or C₁- C₆ alkyl,
or a salt thereof, which comprises,
reacting a compound of formula: 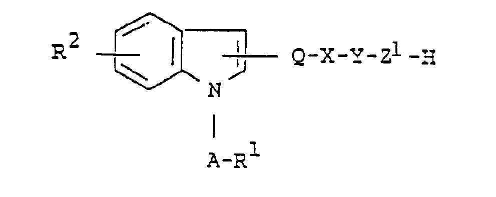 wherein R¹, R², A, Q; X, Y and Z¹ are each as defined above, or a salt thereof, with a compound of the formula: 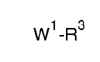 wherein R³ is as defined above, and W¹ is acid residue, or a salt thereof.

5. A process for preparing a compound of the formula: 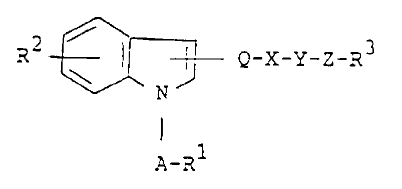
wherein R¹, R², R³, A, Q, X, Y and Z are each as defined in Claim 1 or a salt thereof, which comprises, reacting a compound of the formula: 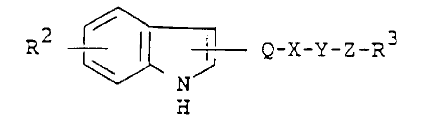
wherein R², R³, Q, X; Y and Z are each as defined above, or a salt thereof, with a compound of the formula: 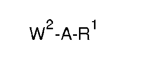
wherein R¹ and A are each as defined above, and W² is acid residue, or a salt thereof.

6. A process for preparing a compound of the formula: 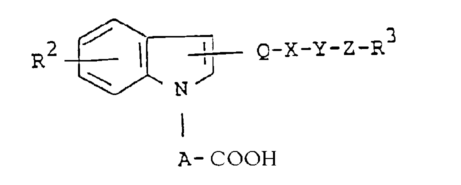
wherein R², R³, A, Q, X, Y and Z are each as defined in claim 1, or a salt thereof, which comprises,
subjecting a compound of the formula: 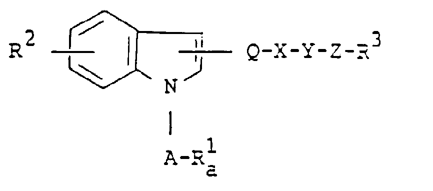 wherein
R², R³, A, Q, X, Y and Z are each as defined above, and
R 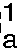 is protected carboxy,
or a salt thereof, to elimination reaction of the carboxy protective group.

7. A pharmaceutical composition comprising a compound of claim 1 or pharmaceutically acceptable salt thereof in association with a pharmaceutically acceptable, substantially non-toxic carrier or excipient.

8. A compound of claim 1 or pharmaceutically acceptable salt thereof for use as a medicament.

9. A compound of claim 1 or pharmaceutically acceptable salt thereof for use as a testosteron 5a-reductase inhibitor.

10. A process for preparing a pharmaceutical composition which comprises admixing a compound of claim 1 or pharmaceutically acceptable salt thereof with a pharmaceutically acceptable, substantially non-toxic carrier or excipient.

11. Use of a compound of claim 1 or pharmaceutically acceptable salt thereof for the manufacture of a medicament for treating alopecia, acnes and prostatism.

## Claims (Claims for the following Contracting State(s) : ES, GR)

1. A process for preparing a compound of the formula: 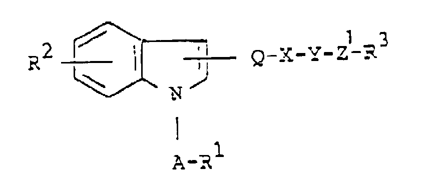 wherein
R¹ is carboxy or protected carboxy,
R² is hydrogen or C₁-C₆ alkyl or halogen,
R³ is mono- or diphenyl (C₁-C₆)-alkyl which may be substituted by C₁-₆ alkyl or 10.11-dihydro-5H-dibenzo [b, f]azepinyl,
A is C₁-C₆ alkylene,
Q is carbonyl,
X is 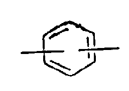
Y is bond,
Z¹ is -O- or 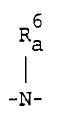
in which R 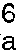 is hydrogen or C₁-C₆ alkyl,
or a salt thereof,
which comprises,
reacting a compound of the formula: 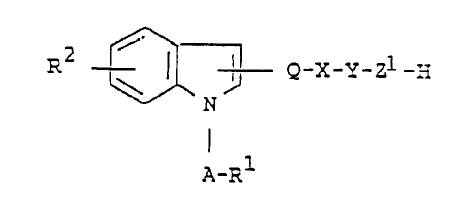
wherein R¹, R², A, Q; X, Y and Z¹ are each as defined above, or a salt thereof, with a compound of the formula: 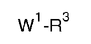 wherein
R³ is as defined above, and
W¹ is acid residue,
or a salt thereof.

2. A process for preparing a compound of the formula: 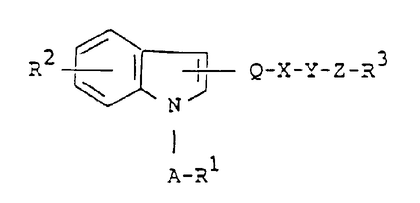 wherein R¹, R², R³, A, Q, X and Y are each as defined in claim 1 Z is C₁-C₆ alkylene, -O- or 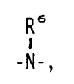
in which R⁶ is hydrogen or C₁-C₆ alkyl or phenyl(C₁-C₆) alkyl
or a salt thereof,
which comprises,
reacting a compound of the formula: 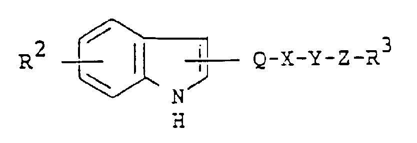
wherein R², R³, Q, X; Y and Z are each as defined above, or a salt thereof, with a compound of the formula: 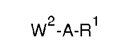 wherein
R¹ and A are each as defined above, and
W² is acid residue,
or a salt thereof.

3. A process for preparing a compound of the formula: 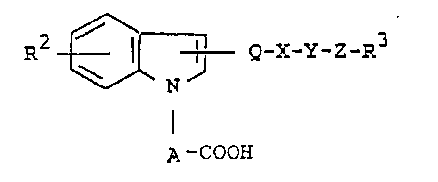
wherein R², R³, A, Q; X, Y and Z are each as defined in claims 1 or 2; or a salt thereof, which comprises,
subjecting a compound of the formula: 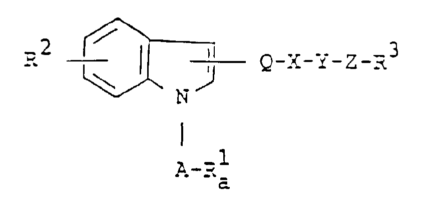 wherein
R², R³, A, Q, X, Y and Z are each as defined above, and
R 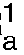 is protected carboxy,
or a salt thereof, to elimination reaction of the carboxy protective group

4. A process for preparing a pharmaceutical composition which comprises admixing a compound obtained according to any of claims 1 to 3 or pharmaceutically acceptable salt thereof with a pharmaceutically acceptable, substantially non-toxic carrier or excipient.

## Patentansprüche (Patentansprüche für folgende(n) Vertragsstaat(en) : AT, BE, CH, DE, DK, FR, GB, IT, LI, LU, NL, SE:)

1. Verbindung der Formel: 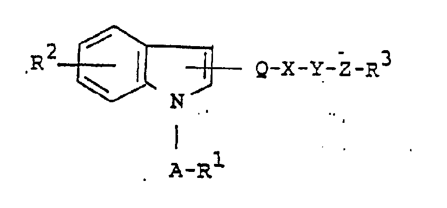 worin
R¹ Carboxy oder geschütztes Carboxy ist,
R² Wasserstoff oder (C₁-C₆)Alkyl oder Halogen ist,
R³ Mono- oder Diphenyl(C₁-C₆)alkyl, das mit (C₁-C₆)Alkyl substituiert sein kann, oder 10,11-Dihydro-5H-dibenzo-[b,f]azepinyl ist,
A (C₁-C₆)Alkylen ist,
Q Carbonyl ist,
X 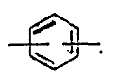
ist
Y eine Bindung ist,
Z (C₁-C₆)Alkylen, -O- oder 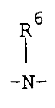
ist, worin R⁶ Wasserstoff,
oder (C₁-C₆)Alkyl oder Phenyl(C₁-C₆)alkyl ist, und pharmazeutisch verträgliche Salze davon.

2. Verbindung nach Anspruch 1, die durch die Formel dargestellt wird: 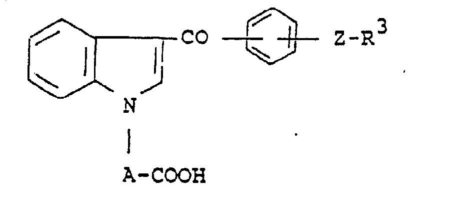 worin R³, A und Z wie in Anspruch 1 definiert sind.

3. Verbindung nach Anspruch 2, die aus der Gruppe ausgewählt wird, die besteht aus:
4-[3-[3-[Bis(4-isobutylphenyl)methylamino]benzoyl]indol-1-yl]butansäure,
4-[3-[4-[Bis(4-isobutylphenyl)methoxy]benzoyl]indol-1 -yl]butansäure,
4-[3-[4-[1 -(4-isobutylphenyl)ethoxy]benzoyl]indol-1 -yl]butansäure und
4-[3-[3-[2,2-Bis(4-isobutylphenyl)ethyl]benzoyl]indol-1-yl]butansäure.

4. Verfahren zur Herstellung einer Verbindung der Formel: 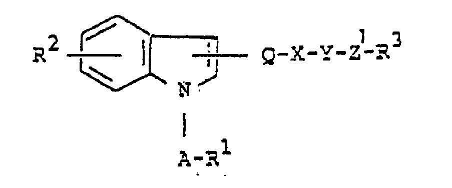 worin R¹, R², R³, A, Q, X und Y jeweils wie in Anspruch 1 definiert sind, Z¹ -O- oder 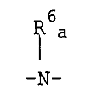 ist, worin R⁶ₐ Wasserstoff oder (C₁-C₆)Alkyl ist, oder eines Salzes davon, das umfaßt: Reagieren einer Verbindung der Formel: 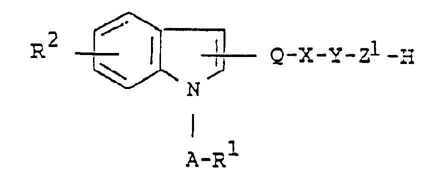 worin R¹, R², A, Q, X; Y und Z¹ jeweils wie oben definiert sind, oder eines Salzes davon mit einer Verbindung der Formel: 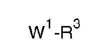 worin R³ wie oben definiert ist und W¹ ein saurer Rest ist, oder einem Salz davon.

5. Verfahren zur Herstellung einer Verbindung der Formel: 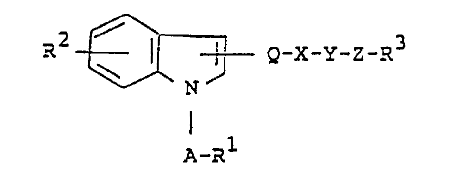 worin R⁷, R², R³, A, Q, X, Y und Z jeweils wie in Anspruch 1 definiert sind, oder eines Salzes davon, das umfaßt: Reagieren einer Verbindung der Formel: 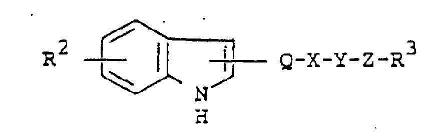 worin R², R³, A, Q, X, Y und Z jeweils wie oben definiert sind, oder eines Salzes davon, mit einer Verbindung der Formel: 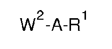 worin R¹ und A jeweils wie oben definiert sind und W² ein saurer Rest ist, oder einem Salz davon.

6. Verfahren zur Herstellung einer Verbindung der Formel: 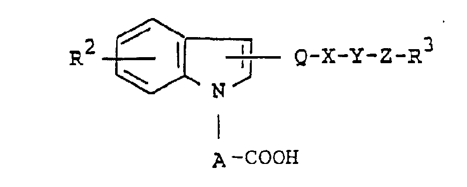
worin R², R³, A, Q, X, Y und Z jeweils wie in Anspruch 1 definiert sind,
oder eines Salzes davon,
das umfaßt:
Unterwerfen einer Verbindung der Formel: 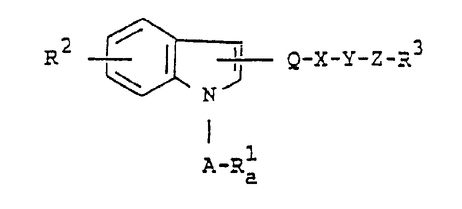
worin R², R³, A, Q, X, Y und Z jeweils wie oben definiert sind und
R^{l}_{"} geschütztes Carboxy ist, oder eines Salzes davon der Eliminierungsreaktion der Carboxyschutzgruppe.

7. Pharmazeutische Zusammensetzung, die eine Verbindung nach Anspruch 1 oder ein pharmazeutisch verträgliches Salz davon in Assoziation mit einem pharmazeutisch verträglichen, im wesentlichen nicht giftigen Träger oder Exzipienten umfaßt.

8. Verbindung nach Anspruch 1 oder ein pharmazeutisch verträgliches Salz davon zur Verwendung als Medikament.

9. Verbindung nach Anspruch 1 oder ein pharmazeutisch verträgliches Salz davon zur Verwendung als Testosteron-5a-Reduktase-Inhibitor.

10. Verfahren zur Herstellung einer pharmazeutischen Zusammensetzung, daß das Zusammenmischen einer Verbindung nach Anspruch 1 oder eines pharmazeutisch verträglichen Salzes davon mit einem pharmazeutisch verträglichen, im wesentlichen nicht giftigen Träger oder Exzipienten umfaßt.

11. Verwendung einer Verbindung nach Anspruch 1 oder eines pharmazeutisch verträglichen Salzes davon zur Herstellung eines Medikamentes zur Behandlung von Alopezie, Akne und Prostatismus.

## Patentansprüche (Patentansprüche für folgende(n) Vertragsstaat(en) : ES, GR)

1. Verfahren zur Herstellung einer Verbindung der Formel: 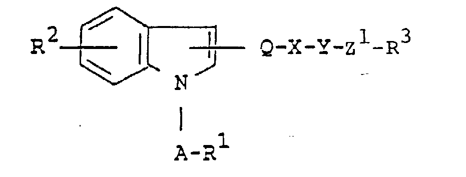 worin
R¹ Carboxy oder geschütztes Carboxy ist,
R² Wasserstoff oder (C₁-C₆)Alkyl oder Halogen ist,
R³ Mono- oder Diphenyl(C₁-C₆)alkyl, das mit (C₁-C₆)Alkyl substituiert sein kann, oder 10,11-Dihydro-5H-dibenzo-[b,f]azepinyl ist,
A (C₁-C₆)Alkylen ist,
Q Carbonyl ist,
X 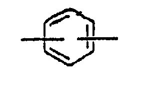
ist
Y eine Bindung ist,
Z¹ (C₁-C₆)Alkylen, -0- oder 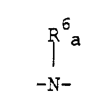
ist, worin R⁶ₐ Wasserstoff,
oder (C₁-C₆)Alkyl ist,
oder eines Salzes davon, das umfaßt:
Reagieren einer Verbindung der Formel: 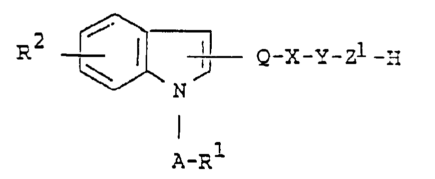
worin R¹, R², A; Q, X, Y und Z¹ jeweils wie oben definiert sind, oder eines Salzes davon mit einer Verbindung der Formel: 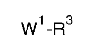
worin R³ wie oben definiert ist und W¹ ein saurer Rest ist, oder einem Salz davon.

2. Verfahren zur Herstellung einer Verbindung der Formel: 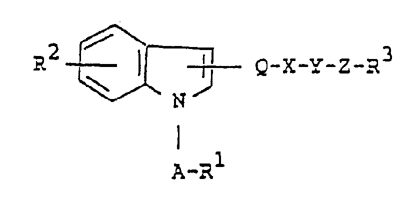
worin R¹, R², R³, A, Q, X, und Y jeweils wie in Anspruch 1 definiert sind, Z (C₁-C₆)Alkylen, -O- oder 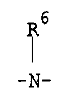
ist, worin R⁶ Wasserstoff, oder (C₁-C₆)Alkyl oder Phenyl(C₁-C₆)alkyl ist, oder eines Salzes davon, das umfaßt:
Reagieren einer Verbindung der Formel: 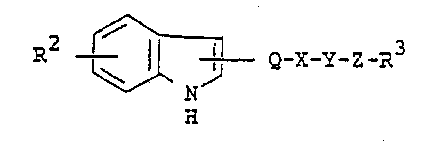
worin R², R³, A, Q, X, Y und Z jeweils wie oben definiert sind, oder eines Salzes davon, mit einer Verbindung der Formel: 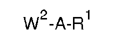
worin R¹ und A jeweils wie oben definiert sind und W² ein saurer Rest ist, oder einem Salz davon.

3. Verfahren zur Herstellung einer Verbindung der Formel: 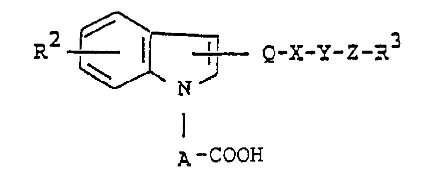
worin R², R³, A, Q, X, Y und Z jeweils wie in Anspruch 1 oder 2 definiert sind, oder eines Salzes davon,
das umfaßt:
Unterwerfen einer Verbindung der Formel: 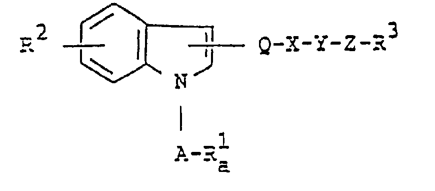
worin R², R³, A, Q, X, Y und Z jeweils wie oben definiert sind und
R¹ₐ geschütztes Carboxy ist, oder eines Salzes davon der Eliminierungsreaktion der Carboxyschutzgruppe.

4. Verfahren zur Herstellung einer pharmazeutischen Zusammensetzung, daß das Zusammenmischen einer Verbindung nach irgendeinem der Ansprüch 1 bis 3 oder eines pharmazeutisch verträglichen Salzes davon mit einem pharmazeutisch verträglichen, im wesentlichen nicht giftigen Träger oder Exzipienten umfaßt

## Revendications (Revendications pour l'(les) Etat(s) contractant(s) suivant(s) : AT, BE, CH, DE, DK, FR, GB, IT, LI, LU, NL, SE)

1. Composé de la formule : 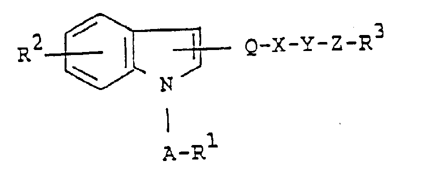 dans laquelle
R¹ est un groupe carboxy ou carboxy protégé,
R² est un atome d'hydrogène ou un groupe alkyle en C₁-C₆ ou un atome d'halogène,
R³ est un groupe mono- ou diphényl(alkyle en C₁-C₆) qui peut être substitué par un groupe alkyle en C₁₋C₆, ou un groupe 10,11-dihydro-5H-dibenzo[b,f]azépinyle,
A est un groupe alkylène en C₁-C₆,
Q est un groupe carbonyle,
X est 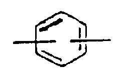
Y est une liaison,
Z est un groupe alkylène en C₁-C₆, -O- ou 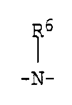
où R⁶ est un atome d'hydrogène ou un groupe alkyle en C₁-C₆ ou un groupe phényl(alkyle en C₁-C₆),
et des sels pharmaceutiquement acceptables de celui-ci.

2. Composé de la revendication 1, qui est représenté par la formule : 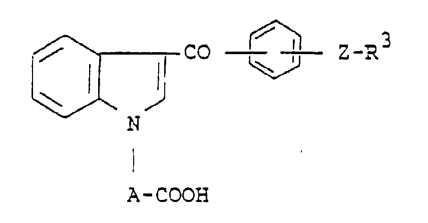 dans laquelle R³, A et Z sont chacun tels que définis en revendication 1.

3. Composé de la revendication 2, qui est choisi dans le groupe constitué des :
Acide 4-[3-[3-[bis(4-isobutylphényl)méthylamino]benzoyl]indol-1-yl)butyrique_{;}
Acide 4-[3-[4-[bis(4-isobutylphényl)méthoxy]benzoyl]indol-1-yl]butyrique,
Acide 4-[3-[4-[1-(4-isobutylphényl)éthoxy]benzoyl]indol-1-yl]butyrique et
Acide 4-[3-[3-[2,2-bis(4-isobutylphényl)éthyl]benzoyl]indol-1-yl]butyrique.

4. Procédé pour préparer un composé de la formule : 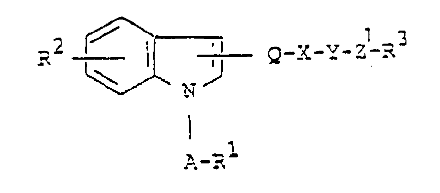 dans laquelle R¹, R², R³, A, Q, X, et Y sont chacun tels que définis en revendication 1. Z¹ est -O- ou 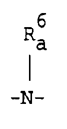
où R 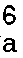 est un atome d'hydrogène ou un groupe alkyle en C₁-C₆,
ou un de ses sels, qui comprend :
la mise à réagir d'un composé de la formule: 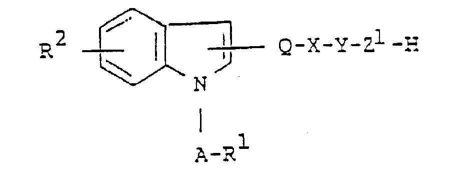
dans laquelle R¹, R², A, Q, X, Y et Z¹ sont chacun tels que définis ci-dessus, ou un de ses sels, avec un composé de la formule : 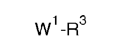
dans laquelle R³ est tel que défini ci-dessus, et W¹ est un résidu d'acide, ou un de ses sels.

5. Procédé pour préparer un composé de la formule : 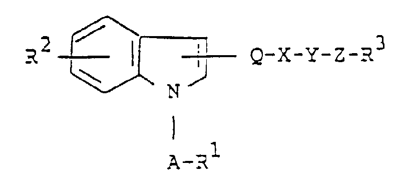 dans laquelle R¹, R², R³, A, Q, X; Y et Z sont chacun tels que définis en revendication 1. ou un de ses sels, qui comprend :
la mise à réagir d'un composé de la formule : 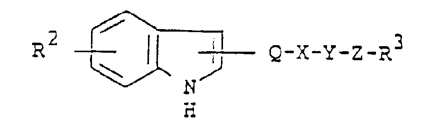
dans laquelle R², R³, Q, X, Y et Z sont chacun tels que définis ci-dessus, ou un de ses sels, avec un composé de la formule : 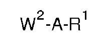
dans laquelle R¹ et A sont chacun tels que définis ci-dessus, et W² est un résidu d'acide, ou un de ses sels.

6. Procédé pour préparer un composé de la formule : 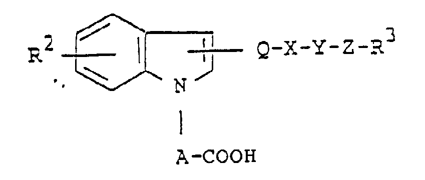 dans laquelle R², R³, A, Q, X, Y et Z sont chacun tels que définis ci-dessus,. ou un de ses sels, qui comprend :
la soumission d'un composé de la formule : 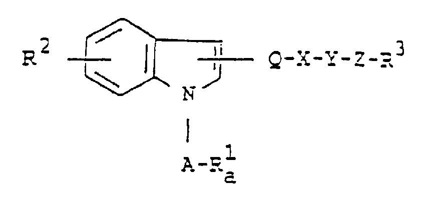
dans laquelle R², R³, A, Q, X, Y et Z sont chacun tels que définis ci-dessus, et R 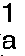 est un groupe carboxy protégé,
ou un de ses sels, à une réaction d'élimination du groupe protecteur du groupe carboxy

7. Composition pharmaceutique comprenant un composé de la revendication 1 ou son sel pharmaceutiquement acceptable en association avec un support ou un excipient pharmaceutiquement acceptable, sensiblement non toxique.

8. Composé selon la revendication 1 ou son sel pharmaceutiquement acceptable pour emploi comme médicament.

9. Composé selon la revendication 1 ou son sel pharmaceutiquement acceptable pour utilisation comme inhibiteur de la 5a-réductase de la testostérone.

10. Procédé pour préparer une composition pharmaceutique qui consiste à mélanger un composé de la revendication 1 ou son sel pharmaceutiquement acceptable avec un support ou un excipient pharmaceutiquement acceptable, sensiblement non toxique.

11. Utilisation d'un composé de la revendication 1 ou de son sel pharmaceutiquement acceptable pour la fabrication d'un médicament pour le traitement de l'alopécie, de l'acné et du prostatisme.

## Revendications (Revendications pour l'(les) Etat(s) contractant(s) suivant(s) : ES, GR)

1. Procédé pour préparer un composé de la formule : 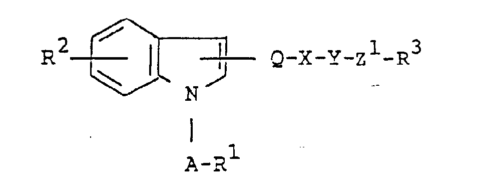 dans laquelle
R¹ est un groupe carboxy ou carboxy protégé,
R² est un atome d'hydrogène ou un groupe alkyle en C₁-C₆ ou un atome d'halogène,
R³ est un groupe mono- ou diphényl(alkyle en C₁-C₆) qui peut être substitué par un groupe alkyle en C₁-C₆, ou un groupe 10,11-dihydro-5H-dibenzo[b,f]azépinyle,
A est un groupe alkylène en C₁-C₆,
Q est un groupe carbonyle,
X est 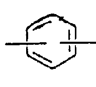
Y est une liaison,
Z est -O- ou 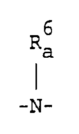
où R 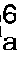 est un atome d'hydrogène ou un groupe alkyle en C₁-C₆,
ou un de ses sels,
qui comprend :
la mise à réagir d'un composé de la formule : 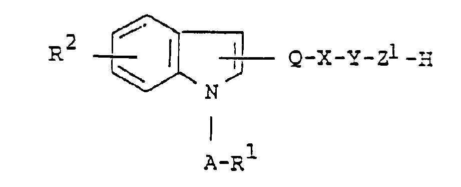
dans laquelle R¹, R², A, Q, X, Y et Z¹ sont chacun tels que définis ci-dessus, ou un de ses sels, avec un composé de la formule : 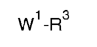
dans laquelle R³ est tel que défini ci-dessus, et W¹ est un résidu d'acide, ou un de ses sels.

2. Procédé pour préparer un composé de la formule : 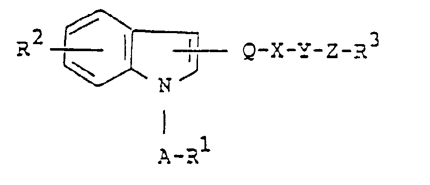 dans laquelle R¹, R², R³, A, Q, X; Y et Z sont chacun tels que définis en revendication 1.
Z est un groupe alkylène en C₁-C₆, -O- ou 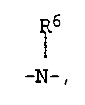
où R⁶ est un atome d'hydrogène ou un groupe alkyle en C₁-C₆ ou phényl(alkyle en C₁-C₆) ou un de ses sels,
qui comprend :
la mise à réagir d'un composé de la formule : 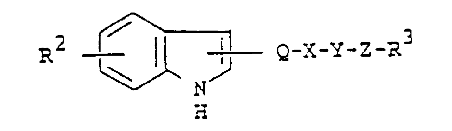
dans laquelle R², R³, Q, X, Y et Z sont chacun tels que définis ci-dessus, ou un de ses sels, avec un composé de la formule : 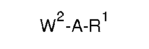
dans laquelle R¹ et A sont chacun tels que définis ci-dessus, et
W² est un résidu d'acide,
ou un de ses sels.

3. Procédé pour préparer un composé de la formule : 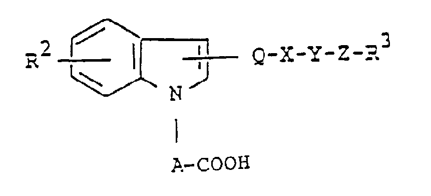 dans laquelle R², R³, A, Q, X, Y et Z sont chacun tels que en revendication 1 ou en revendication 2, ou un de ses sels,
qui comprend :
la soumission d'un composé de la formule : 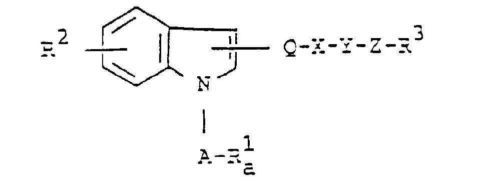
dans laquelle R², R³, A, Q, X, Y et Z sont chacun tels que définis ci-dessus, et R 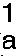 est un groupe carboxy protégé,
ou un de ses sels, à une réaction d'élimination du groupe protecteur du groupe carboxy

4. Procédé pour préparer une composition pharmaceutique qui comprend le mélange d'un composé obtenu conformément à l'une quelconque des revendications 1 à 3 ou son sel pharmaceutiquement acceptable avec un support ou un excipient pharmaceutiquement acceptable, sensiblement non toxique.
